# EUROPEAN PATENT APPLICATION

(11) **EP 4 660 204 A1**
(43) Date of publication of application: **10.12.2025**
(21) Application number: 25180134.6
(22) Date of filing: 02.06.2025
(51) Int. Cl.: C07K 16/28, A61P 13/12

(54) **ANTAGONIST FOR USE IN TREATMENT OF MEMBRANOUS NEPHROPATHY**

(30) Priority: 07.06.2024 US 202463657412 P
(71) Applicant: Chang Gung University, Taoyuan County 33302, Taiwan (TW)
(72) Inventor: WU, Tsai-Yi, 333 Taoyuan City (TW); TU, Kun-Hua, 333 Taoyuan City (TW); KU, Cheng-Lung, 333 Taoyuan City (TW); SHIH, Han-Po, 333 Taoyuan City (TW)
(74) Representative: Viering, Jentschura & Partner mbB Patent- und Rechtsanwälte

(57) **Abstract**

An antagonist for treating membranous nephropathy and use of the antagonist in the manufacture of a pharmaceutical composition for treating the membranous nephropathy. The antagonist is an antibody that is configured to bind to a C-type lectin-like domain 1 (CTLD1) domain or a cysteine-rich (CysR) domain of a phospholipase A2 receptor (PLA2R), or is a recombinant protein that binds to an anti-PLA2R autoantibody.

## Description

### FIELD OF THE INVENTION

The present invention relates to an antagonist and a use thereof, and more particularly to an antagonist for treating membranous nephropathy and a use thereof.

### BACKGROUND OF THE INVENTION

Membranous nephropathy (MN) is a glomerular disease that is organ-specific and immune-mediated, and is characterized by subepithelial deposition of immune complexes in glomeruli. Accumulation of immunoglobulin G (IgG) and complement proteins contributes to thickening of a glomerular capillary wall and podocyte injury (Glassock RJ. Am J Kidney Dis. 2010;56(1):157-167). About 20% of membranous nephropathy cases are related to systemic lupus erythematosus (SLE), hepatitis B virus, malignant tumor, or contact infections and medications, and are thus classified as secondary membranous nephropathy (Ronco P, et al. Membranous nephropathy. Nature Reviews Disease Primers. 2021;7(1):69). However, up to 80% of the membranous nephropathy cases are classified as primary membranous nephropathy (pMN). The primary membranous nephropathy is caused by autoantibodies targeting podocyte plasma membranes. Furthermore, about 75% of cases are related to phospholipase A2 receptor (PLA2R) antigens.

Formation of a PLA2R protein begins with an N-terminal cysteine-rich (CysR) domain, followed by a fibronectin type II (FNII) domain and eight C-type lectin-like domains (CTLD1 to CTLD8) that are different from each other (Dong Y, et al. Journal of Molecular Biology. 2017;429(24):3825-3835). A phospholipase A2 receptor (PLA2R) is present in podocytes, neutrophils (Silliman CC, et al. AJP Cell Physiology. 2002;283(4):C1102-13), and respiratory epithelial cells (Granata F, et al. J Immunol. 2005;174(1):464-74). The PLA2R binds to Group IB secretory phospholipase A2 with high affinity, and may act as signaling molecules to induce the podocyte injury (Pan Y, et al. Scientific Reports. 2014;4, and Yang L, et al. The FASEB Journal. 2021;35(2)). However, specific functions thereof remain to be unclear.

Apart from anti-PLA2R autoantibodies, an expression level of the PLA2R protein will also be increased in pMN patients. However, there is no significant difference in a podocyte-related messenger ribonucleic acid (mRNA) level (Hoxha E, et al. Kidney International. 2012;82(7):797-804). Moreover, it can be observed from a transgenic mouse model having podocyte-specific overexpression of PLA2R that PLA2R expression and presence of the anti-PLA2R autoantibodies may lead to proteinuria (Meyer-Schwesinger C, et al. Kidney International. 2020;97(5):913-919; Tomas NM, et al. Kidney International. 2022;103(2):297-303; and Tomas NM, et al. Kidney International. 2023;104(5): 916-928).

The podocyte injury induced by the anti-PLA2R autoantibodies is already considered to be related to complement activation. Results obtained by adopting immunohistochemistry and mass spectrometry to perform a patient biopsy and a serological analysis show that a series of complement components (which include C3, C4d, C3d, C1q, factor B, a mannose-binding lectin (MBL), a membrane attack complex (C5b-9), etc.) will be activated in the pMN patients (Hayashi N, et al. Nephrology Dialysis Transplantation. 2018;33(5):832-840; Ravindran A, et al. Kidney International Reports. 2020;5(5):618-626; Segawa Y, et al. Pediatric Nephrology. 2010;25(6):1091-1099; and Zhang M, et al. BMC Nephrology. 2019;20(1):313). Moreover, an increased expression level of C5b-9 is already proposed to be taken as a marker of illness severity (Koopman JJE, et al. Frontiers in Immunology. 2021;11). The increased presence of C5b-9 in the glomeruli and urine plays a critical role in the pathogenesis of complement-activated pMN.

The current treatments for the membranous nephropathy include use of non-immunosuppressive drugs (e.g., statins), targeted immunosuppressive drugs (e.g., rituximab), and a conventional immunosuppressive therapy (e.g., cyclophosphamide combined with steroids), etc. (Ronco P, et al. Journal of Clinical Medicine. 2021;10(4):607). However, these treatments are not therapeutic for all patients, and may produce negative side effects. For example, the pMN patients of a high titer group are likely to be unresponsive to the rituximab of low dosage.

Therefore, novel treatment strategies or medications for the membranous nephropathy, and particularly the primary membranous nephropathy, are still needed in the relevant industry.

### SUMMARY OF THE INVENTION

In response to the above-referenced technical inadequacies, the present invention provides a novel treatment strategy and a novel pharmaceutical composition for treating membranous nephropathy.

In order to solve the above-mentioned problems, one of the technical aspects adopted by the present invention is to provide use of an antagonist in the manufacture of a pharmaceutical composition for treating membranous nephropathy. The membranous nephropathy is an autoantibody-mediated membranous nephropathy. The antagonist is an anti-phospholipase A2 receptor (PLA2R) antibody, and is configured to bind to a C-type lectin-like domain 1 (CTLD1) domain or a cysteine-rich (CysR) domain of a phospholipase A2 receptor (PLA2R).

In one of the possible or preferred embodiments, the anti-PLA2R antibody contains light chain complementarity-determining regions L-CDR1, L-CDR2, and L-CDR3, and heavy chain complementarity-determining regions H-CDR1, H-CDR2, and H-CDR3.

In one of the possible or preferred embodiments, the L-CDR1, the L-CDR2, and the L-CDR3 of the anti-PLA2R antibody respectively contain amino acid sequences set forth in SEQ ID NOs: 151, 152, and 153, and the H-CDR1, the H-CDR2, and the H-CDR3 respectively contain amino acid sequences set forth in SEQ ID NOs: 112, 113, and 114.

In one of the possible or preferred embodiments, the L-CDR1, the L-CDR2, and the L-CDR3 of the anti-PLA2R antibody respectively contain amino acid sequences set forth in SEQ ID NOs: 154, 155, and 156, and the H-CDR1, the H-CDR2, and the H-CDR3 respectively contain amino acid sequences set forth in SEQ ID NOs: 115, 116, and 117.

In one of the possible or preferred embodiments, the L-CDR1, the L-CDR2, and the L-CDR3 of the anti-PLA2R antibody respectively contain amino acid sequences set forth in SEQ ID NOs: 157, 158, and 159, and the H-CDR1, the H-CDR2, and the H-CDR3 respectively contain amino acid sequences set forth in SEQ ID NOs: 118, 119, and 120.

In one of the possible or preferred embodiments, the L-CDR1, the L-CDR2, and the L-CDR3 of the anti-PLA2R antibody respectively contain amino acid sequences set forth in SEQ ID NOs: 160, 161, and 162, and the H-CDR1, the H-CDR2, and the H-CDR3 respectively contain amino acid sequences set forth in SEQ ID NOs: 121, 122, and 123.

In one of the possible or preferred embodiments, the L-CDR1, the L-CDR2, and the L-CDR3 of the anti-PLA2R antibody respectively contain amino acid sequences set forth in SEQ ID NOs: 163, 164, and 165, and the H-CDR1, the H-CDR2, and the H-CDR3 respectively contain amino acid sequences set forth in SEQ ID NOs: 124, 125, and 126.

In one of the possible or preferred embodiments, the L-CDR1, the L-CDR2, and the L-CDR3 of the anti-PLA2R antibody respectively contain amino acid sequences set forth in SEQ ID NOs: 166, 167, and 168, and the H-CDR1, the H-CDR2, and the H-CDR3 respectively contain amino acid sequences set forth in SEQ ID NOs: 127, 128, and 129.

In one of the possible or preferred embodiments, the L-CDR1, the L-CDR2, and the L-CDR3 of the anti-PLA2R antibody respectively contain amino acid sequences set forth in SEQ ID NOs: 169, 170, and 171, and the H-CDR1, the H-CDR2, and the H-CDR3 respectively contain amino acid sequences set forth in SEQ ID NOs: 130, 131, and 132.

In one of the possible or preferred embodiments, the L-CDR1, the L-CDR2, and the L-CDR3 of the anti-PLA2R antibody respectively contain amino acid sequences set forth in SEQ ID NOs: 172, 173, and 174, and the H-CDR1, the H-CDR2, and the H-CDR3 respectively contain amino acid sequences set forth in SEQ ID NOs: 133, 134, and 135.

In one of the possible or preferred embodiments, the L-CDR1, the L-CDR2, and the L-CDR3 of the anti-PLA2R antibody respectively contain amino acid sequences set forth in SEQ ID NOs: 175, 176, and 177, and the H-CDR1, the H-CDR2, and the H-CDR3 respectively contain amino acid sequences set forth in SEQ ID NOs: 136, 137, and 138.

In one of the possible or preferred embodiments, the L-CDR1, the L-CDR2, and the L-CDR3 of the anti-PLA2R antibody respectively contain amino acid sequences set forth in SEQ ID NOs: 178, 179, and 180, and the H-CDR1, the H-CDR2, and the H-CDR3 respectively contain amino acid sequences set forth in SEQ ID NOs: 139, 140, and 141.

In one of the possible or preferred embodiments, the L-CDR1, the L-CDR2, and the L-CDR3 of the anti-PLA2R antibody respectively contain amino acid sequences set forth in SEQ ID NOs: 181, 182, and 183, and the H-CDR1, the H-CDR2, and the H-CDR3 respectively contain amino acid sequences set forth in SEQ ID NOs: 142, 143, and 144.

In one of the possible or preferred embodiments, the L-CDR1, the L-CDR2, and the L-CDR3 of the anti-PLA2R antibody respectively contain amino acid sequences set forth in SEQ ID NOs: 184, 185, and 186, and the H-CDR1, the H-CDR2, and the H-CDR3 respectively contain amino acid sequences set forth in SEQ ID NOs: 145, 146, and 147.

In one of the possible or preferred embodiments, the L-CDR1, the L-CDR2, and the L-CDR3 of the anti-PLA2R antibody respectively contain amino acid sequences set forth in SEQ ID NOs: 187, 188, and 189, and the H-CDR1, the H-CDR2, and the H-CDR3 respectively contain amino acid sequences set forth in SEQ ID NOs: 148, 149, and 150.

In one of the possible or preferred embodiments, the anti-PLA2R antibody is a monoclonal antibody or a recombinant antibody. In other embodiments, antibody fragments of the above-mentioned antibody include a fragment antigen-binding (Fab) fragment, an F(ab')2 fragment, an Fab' fragment, etc.

In one of the possible or preferred embodiments, the anti-PLA2R antibody is an antibody in which a fragment crystallizable (Fc) fragment is inactivated.

In order to solve the above-mentioned problems, another one of the technical aspects adopted by the present invention is to provide use of an antagonist in the manufacture of a pharmaceutical composition for treating membranous nephropathy. The membranous nephropathy is an autoantibody-mediated membranous nephropathy. The antagonist is a recombinant protein, and is configured to bind to an anti-PLA2R autoantibody.

In one of the possible or preferred embodiments, the recombinant protein contains a peptide encoded by a nucleic acid sequence set forth in SEQ ID NO: 190 or 191.

In order to solve the above-mentioned problems, yet another one of the technical aspects adopted by the present invention is to provide an antagonist for treating membranous nephropathy. The antagonist for treating the membranous nephropathy includes the antagonist as mentioned above, and the membranous nephropathy is the autoantibody-mediated membranous nephropathy.

Therefore, the novel treatment strategy and the novel pharmaceutical composition for treating the membranous nephropathy are provided to address the problems in the conventional technology. After much experimentation, it is observed that the anti-PLA2R autoantibody needs to bind to the CTLD1 domain or the CysR domain of the PLA2R, and relies on the Fc fragment of immunoglobulin G1 (IgG1) or the Fc fragment of IgG3 to induce complement-dependent cytotoxicity (CDC).

Accordingly, the novel treatment strategy for the membranous nephropathy provided in the present invention includes using the antagonist to interfere with binding of the anti-PLA2R autoantibody and the PLA2R in cells, so as to suppress cytotoxicity of the anti-PLA2R autoantibody.

In the present invention, by virtue of "the antagonist being the antibody and being configured to bind to the CTLD1 domain or the CysR domain of the PLA2R" and/or "the antagonist being the recombinant protein and being configured to bind to the anti-PLA2R autoantibody," the antagonist can block binding of the anti-PLA2R autoantibody and the PLA2R in the cells (e.g., podocytes), thereby blocking the complement-dependent cytotoxicity induced by the anti-PLA2R autoantibody and reducing cell damage.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 shows an enzyme-linked immunosorbent assay (ELISA) analysis result of specificity of anti-PLA2R monoclonal antibodies;
FIG. 2 shows a western blot analysis result of the specificity of the anti-PLA2R monoclonal antibodies;
FIG. 3 shows analysis results of the specificity of the anti-PLA2R monoclonal antibodies by use of cell fluorescence staining and a flow cytometer;
FIG. 4 and FIG. 5 show analysis results of binding epitopes of the anti-PLA2R monoclonal antibodies;
FIG. 6 shows an affinity analysis result of the anti-PLA2R monoclonal antibodies;
FIG. 7 shows an analysis result of antigenic epitope binning of the anti-PLA2R monoclonal antibodies;
FIG. 8 to FIG. 13 show a set of activity analysis results of the anti-PLA2R monoclonal antibodies of different IgG subclasses for inducing CDC;
FIG. 14, FIG. 15A, and FIG. 15B show another set of activity analysis results of the anti-PLA2R monoclonal antibodies of different IgG subclasses for inducing the CDC;
FIG. 16 shows an analysis result of using an Fab fragment of the anti-PLA2R monoclonal antibody to suppress CDC induction; and
FIG. 17 shows an analysis result of using a CTLD1 recombinant protein and a CysR recombinant protein to suppress the CDC induction.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

Unless otherwise stated, the material(s) used in any described embodiment is/are commercially available material(s) or may be prepared by methods known in the related art, and the process(es) or operation(s) involved in any described embodiment is/are conventional process(es) or operation(s) generally known in the related art.

The term "protein" as used herein refers to a biomacromolecule formed by one or multiple chains of amino acid residues.

The term "antibody" and "immunoglobulin" have the same meaning, and are used as equivalents in the present invention. The term "antibody" as used herein refers to immunoglobulin molecules and immunologically active portions of the immunoglobulin molecules (i.e., molecules that contain an antigen-binding site for specifically binding an antigen). As such, the term "antibody" not only covers a complete antibody molecule but also includes an antibody fragment or derivative.

An antibody includes, but is not limited to, a monoclonal antibody, a polyclonal antibody, a monospecific antibody, a multispecific antibody (e.g., a bispecific antibody), a monovalent antibody, a multivalent antibody, a complete antibody, a fragment of the complete antibody, a naked antibody, a conjugated antibody, a chimeric antibody, a humanized antibody, or a fully human antibody.

In a natural antibody, two heavy chains are connected by a disulfide bond, and each heavy chain is connected to a light chain by the disulfide bond. There are two types of the light chain: a lambda (λ) chain and a kappa (κ) chain. There are mainly five types (isotypes) of the heavy chain that determine functional activity of an antibody molecule: IgM, IgD, IgG, IgA, and IgE. Each chain contains different sequence domains. The light chain contains two domains, i.e., a variable domain (VL) and a constant domain (CL). The heavy chain contains four domains, i.e., one variable domain (VH) and three constant domains (CH1, CH2, and CH3 that are collectively referred to as CH). The variable domain (VL) of the light chain and the variable domain (VH) of the heavy chain can determine binding recognition and specificity to the antigen. The constant domain (CL) of the light chain and the constant domains (CH) of the heavy chain have important biological properties, such as antibody chain binding, secretion, trans-placental mobility, complement binding, and binding to an Fc receptor (FcR). A fragment variable (Fv) fragment is an N-terminal part of an Fab fragment of an immunoglobulin, and is formed by variable portions of one light chain and one heavy chain. Specificity of the antibody resides in structural complementarity between an antibody binding site and an antigenic determinant. The antibody binding site is formed by residues that are primarily from a hypervariable region or a complementarity-determining region (CDR). Occasionally, residues from a non-hypervariable region or a framework region (FR) may affect an overall domain structure, thereby affecting a binding site. The CDR refers to amino acid sequences that jointly define binding affinity and specificity of a natural Fv region at a native immunoglobulin binding site. The light chain of the immunoglobulin has three CDRs (which are designated as L-CDR1, L-CDR2, and L-CDR3), and the heavy chain of the immunoglobulin has three CDRs (which are designated as H-CDR1, H-CDR2, and H-CDR3). As such, the antigen-binding site conventionally contains six CDRs, which include respective CDR sets of a heavy chain V region and a light chain V region. The framework region (FR) refers to amino acid sequences inserted between the CDRs.

The term "monoclonal antibody (mAb)" as used herein refers to an antibody composition having a homogeneous antibody population that binds same epitopes. The above-mentioned term is not limited by antibody types or sources, and is also not limited by preparation processes thereof. Hence, the above-mentioned term covers an antibody obtained by hybridoma technology in mice or other animals, and a human monoclonal antibody obtained by the hybridoma technology in humans (not mice). The above-mentioned term also covers antibodies obtained by other known processes for producing the monoclonal antibody in the conventional technology, such as eukaryotic cell lines produced by transient or stable transfection.

The term "fragment antigen-binding (Fab) fragment" refers to an antibody fragment that contains the constant domain and the variable domain of each of the heavy chain and the light chain. The variable domain contains the antigen-binding site. Conventionally, the antibody contains a fragment crystallizable (Fc) region and two Fab fragments. The Fab fragment can be isolated from the Fc region to generate the two Fab fragments (otherwise referred to as a F(ab')2 fragment or a dimeric antigen-binding fragment).

The term "vector" as used herein refers to a nucleic acid that can be used for introducing another linked nucleic acid into a cell. One type of the vector is a "plasmid", which refers to a linear or circular double stranded DNA molecule that can be ligated with additional nucleic acid segments. Another type of the vector is a viral vector (e.g., replication defective retroviruses, adenoviruses, and adeno-associated viruses), in which additional DNA segments can be introduced into a viral genome. Certain vectors are capable of autonomous replication when being introduced into a host cell (e.g., a bacterial vector containing a bacterial origin of replication and an episomal mammalian vector). Other vectors (e.g., a non-episomal mammalian vector) are integrated into a genome of the host cell upon introduction into the host cell, and are thereby replicated along with the host genome.

The term "specific binding" as used herein refers to an antigen-binding molecule (e.g., the antibody) typically using high affinity to specifically bind the antigen and a substantially identical antigen, and not using high affinity to bind unrelated antigens. Affinity is typically reflected by an equilibrium dissociation constant (K_{D}), where a lower K_{D} indicates higher affinity. Taking the antibody as an example, the high affinity typically indicates having a K_{D} of about 1 × 10⁻⁷ M or less, about 1 × 10⁻⁸ M or less, about 1 × 10⁻⁹ M or less, about 1 × 10⁻¹⁰ M or less, 1 × 10⁻¹¹ M or less, or 1 × 10⁻¹² M or less. The equilibrium dissociation constant is calculated as follows: K_{D} = K_{d}/Kₐ, where K_{d} represents a dissociation rate and Kₐ represents a binding rate. The equilibrium dissociation constant can be measured by using well-known methods in the related art, such as surface plasmon resonance (e.g., Biacore) or equilibrium dialysis.

The term "treatment" as used herein refers to a surgical or therapeutic treatment for the purpose of preventing or slowing (reducing) an undesired physiological or pathological change (e.g., progression of a cancer) in a treated subject. Beneficial or desired clinical results include, but are not limited to, alleviation of symptoms, a decrease in severity of illness, stabilization (i.e., not worsening) of a state of illness, delay or slowing of illness progression, amelioration or palliation of the state of illness, and remission of the state of illness (whether partial or complete and whether detectable or undetectable). Subjects in need of treatment include those already with a disorder or illness, as well as those who are susceptible to a disorder or illness or those who intend to prevent a disorder or illness. When referring to terms such as slowing, alleviation, decrease, palliation, and remission, their meanings also include elimination, disappearance, nonoccurrence, etc.

The term "subject" as used herein refers to an organism that receives treatment for a particular illness or disorder mentioned in the present invention. Examples of subjects and patients include mammals, such as human, primates (e.g., monkeys), or non-primates, that receive treatment for an illness or disorder.

The term "effective amount" as used herein refers to an amount of a therapeutic agent that is effective in preventing or alleviating symptoms of an illness or progression of the illness when administered to a cell, tissue, or subject alone or in combination with another therapeutic agent. The effective amount also refers to an amount of a compound that is sufficient to alleviate symptoms (e.g., to treat, cure, prevent, or alleviate related medical disorders), or to increase rates at which such disorders are treated, cured, prevented, or alleviated. When an active ingredient is administered alone to an individual, a therapeutically effective dose refers to an amount of the ingredient alone. When a combination is used, the therapeutically effective dose refers to a combined amount of multiple active ingredients that produce a therapeutic effect (whether administered in combination, sequentially, or simultaneously).

In the following descriptions, advantages and features of the present invention will be discussed in detail in conjunction with specific examples. Under the circumstance where specific conditions are not indicated, the examples are carried out according to conventional conditions or according to conditions recommended by a manufacturer. It should be noted that reagents or instruments used in the examples of the present invention can be replaced by commercially available products of the same nature. Unless indicated otherwise, cells, vectors, and proteins used for verifying efficacy and activity in the examples of the present invention can all be replaced by commercially available products of the same nature or produced by existing techniques.

### [Embodiment 1 Construction and Purification of Anti-PLA2R Monoclonal Antibody]

### 1.1 Isolation of Single Human B Cells.

Fluorescence-activated cell sorting (FACS) is adopted to isolate the single human B cells.

Specifically, peripheral venous blood samples are collected from four membranous nephropathy (MN) patients. Then, Ficoll-Paque that is currently available on the market (brand: GE Healthcare) is used to perform density gradient centrifugation for purification, and mononuclear cells can be isolated from the peripheral venous blood samples. At a concentration of 1 x 10⁷ cells/ml, the isolated mononuclear cells are resuspended in an FACS buffer (containing 1% of a fetal bovine serum (FBS) and a 2 mM ethylenediaminetetraacetic acid (EDTA)/phosphate-buffered saline (PBS) buffer) with 5% of a normal mouse serum (brand: Jackson ImmunoResearch), and are incubated on ice for 30 minutes. After the mononuclear cells are washed with the FACS buffer, 1 x 10⁶ mononuclear cells are added into 5 µg of a PLA2R protein and incubated on ice for 30 minutes. Before cell sorting, BV421 anti-human IgG (brand: BD Biosciences), FITC anti-human IgG (brand: BD Biosciences), PE/Cyanine7 anti-human CD3 (brand: eBiosciences), anti-human CD19 APC-eFluor 780 (brand: eBiosciences), anti-Myc PE (brand: R&D Systems), and 7-Aminoactinomycin D (brand: Sigma) are used to dye the mononuclear cells for 30 minutes on ice. During the cell sorting, a gating strategy to sort the single human B cells that can bind to the PLA2R protein is: CD19+IgG+CD3-IgD-PE+. The cells are sorted into a 96-well plate that carries 18 µl/well of an RT-lysis buffer. After the cell sorting is completed, the 96-well plate is sealed with an aluminum sealing tape (brand: Corning) and stored at -80°C.

### 1.2 Synthesis of Complementary DNA (cDNA) of Single Human B Cells

The cDNA of the single human B cells can be synthesized by reverse transcription-polymerase chain reaction (RT-PCR).

Specifically, total ribonucleic acid (RNA) of the above-mentioned single human B cells (which are respectively designated as Cell-1B10, Cell-2B8, Cell-1E6, Cell-1E9, Cell-1E12, Cell-1B7, Cell-1D1, Cell-1G5, Cell-2F1, Cell-2F10, Cell-2G5, Cell-2G7, and Cell-2G11) is obtained and subjected to the RT-PCR. For the RT-PCR, 200 ng of a random hexamer primer (brand: Thermo Scientific), 1 µl of a 10 mM dNTP mix (brand: Thermo Scientific). 0.5% (v/v) of a non-ionic surfactant (IGEPAL CA-630; brand: Sigma), 40 U of a Ribolock ribonuclease inhibitor (brand: Fermentas), and 50 U of a Maxima H minus reverse transcriptase (brand: Thermo Scientific) are used. Reaction conditions are as follows: annealing at 42°C for 10 minutes, performing pre-primer extension at 25°C for 10 minutes, performing polymerization at 50°C for 45 minutes, letting stand at 85°C for 5 minutes to achieve enzyme inactivation, and storing the obtained cDNA at -80°C.

### 1.3 Ig V Gene Amplification of Single Human B Cells

A nested polymerase chain reaction (nested PCR) is performed to amplify IgH V, Igλ V, and Igκ V gene transcripts of the obtained single human B cells.

2.5 µl of the above-mentioned cDNA is used as a template to undergo a first round of PCR. Then, 2.5 µl of a product (unpurified) from the first round of PCR is used as a template to undergo a second round of PCR. All rounds of PCR are performed in a total volume of 25 µl, which includes 0.5 µM of a primer mix, 200 µM of dNTP (brand: Thermo Scientific), and 0.5 U of DNA polymerase (Phusion high-fidelity DNA polymerase with an error rate of about 4.4 x 10⁻⁷; brand: Thermo Scientific). A number assigned to each primer used in the PCR is shown in Table 1, and a sequence of the primer under each assigned number is shown in Table 2. All rounds of PCR are performed with diethyl pyrocarbonate (DEPC) treated water. The first and second rounds of PCR are each performed for 35 cycles at 98°C for 10 seconds, at 65°C for 15 seconds, and at 72°C for 30 seconds.

**Table 1**

| Nested PCR | Primers | IgH V gene | Igκ V gene | Igλ V gene |
|---|---|---|---|---|
| First round | Forward primer (SEQ ID No.) | 1-13 | 24-31 | 40-51 |
| | Reverse primer (SEQ ID No.) | 63 | 65 | 67 |
| Second round | Forward primer (SEQ ID No.) | 14-23 | 32-39 | 52-62 |
| | Reverse primer (SEQ ID No.) | 64 | 66 | 68 |

**Table 2**

| Forward Primer | 5'-3' Sequence | SEQ ID No. |
|---|---|---|
| VH1/7L | accatggactgsacctggag | 1 |
| VH2L | caccatggacacactttgctmcac | 2 |
| VH2-70L | accatggacatactttgttccacg | 3 |
| VH3L | atggagtttgggctgagctg | 4 |
| VH3-21L | atggaactggggctccgctg | 5 |
| VH3-48L | atggagttggggctgtgctg | 6 |
| VH3-49L | catggagtttgggcttagctg | 7 |
| VH3-53L | catggagttttggctgagctg | 8 |
| VH4L | catgaaacacctgtggttcttcct | 9 |
| VH4-39L | aatgaagcacctgtggttcttcct | 10 |
| VH4-59L | acatgaaacatctgtggttcttcct | 11 |
| VH5L | atggggtcaaccgccatcct | 12 |
| VH6L | aatgtctgtctccttcctcatcttcct | 13 |
| VH1/3/5f | saggtgcagctggtgsagtc | 14 |
| VH1-3f | caggtccagcttgtgcagtc | 15 |
| VH1-18f | caggttcagctggtgcagtc | 16 |
| VH1-24f | caggtccagctggtacagtctg | 17 |
| VH2f | caggtcacctgarggagtctggt | 18 |
| VH3-23f | gaggtgcagctgttggagtct | 19 |
| VH4f | cagstgcagctgcaggagt | 20 |
| VH4-34f | caggtgcagctacarcagtgg | 21 |
| VH6f | caggtacagctgcagcagtca | 22 |
| VH7f | caggtgcagctggtgcaat | 23 |
| KV1L | ggtccccgctcagctcctgg | 24 |
| KV1-16L | agtcctcgctcagctcctgg | 25 |
| KV2L | gctccctgctcagctcctgg | 26 |
| KV2-24L | gctccttgctcagcttctgg | 27 |
| KV3L | cctgctactctggctcccag | 28 |
| KV4L | atttctctgttgctctggatctctg | 29 |
| KV5L | cttcctcctcctttggatctctg | 30 |
| KV6L | tctgctgctctgggttccag | 31 |
| VK1f | gacatccagwtgacccagtctcc | 32 |
| VK2f | gatattgtgatgacccagactccactct | 33 |
| VK2-28f | gatattgtgatgactcagtctccactct | 34 |
| VK3f | gaaattgtgttgacrcagtctccag | 35 |
| VK3-15f | gaaatagtgatgacgcagtctccag | 36 |
| VK4f | gacatcgtgatgacccagtctc | 37 |
| VK5f | gaaacgacactcacgcagtctc | 38 |
| VK6f | gaaattgtgctgactcagtctcca | 39 |
| LV1L | ggtcctgggcccagtctgtg | 40 |
| LV2L | ggtcctgggcycagtctgcc | 41 |
| LV3L | gctctgwggcctcctatgagct | 42 |
| LV3-12/21L | gctctgtgacctcctatgwgctg | 43 |
| LV3-19L | gttctgtggtttcttctgagctgact | 44 |
| LV4L | ggtctctctcccwgcytgtgc | 45 |
| LV5L | gttccctctcgcaggctgtg | 46 |
| LV5/9L | gktccctctcccagcctgtg | 47 |
| LV6L | gttcttgggccaattttatgctg | 48 |
| LV7L | ggtccaattcycagrctgtggtg | 49 |
| LV8L | gagtggattctcagactgtggtga | 50 |
| LV10L | tgtcagtggtccaggcaggg | 51 |
| LV1-40/50/51f | cagtctgtgytgacgcagcc | 52 |
| LV1-36/44/47f | cagtctgtgctgactcagcca | 53 |
| LV2f | cagtctgccctgactcagcc | 54 |
| LV3f | tcctatgagctgacwcagcca | 55 |
| LV3-19f | tcttctgagctgactcaggacc | 56 |
| LV4/5/9f | cagsctgtgctgactcagcc | 57 |
| LV4-60f | cagcctgtgctgactcaatcat | 58 |
| LV4-69f | cagcttgtgctgactcaatcg | 59 |
| LV6f | aattttatgctgactcagccccac | 60 |
| LV7/8f | cagrctgtggtgacycaggagc | 61 |
| LV10f | caggcagggctgactcagcc | 62 |

| Reverse Primer | 5'-3' Sequence | |
|---|---|---|
| CγCH1-1 | aggtgtgcacgccgctggtc | 63 |
| CγCH1-2 | ggttcggggaagtagtccttgac | 64 |
| Cκ543-566 | gtttctcgtagtctgctttgctca | 65 |
| Cκ494-516 | gtgctgtccttgctgtcctgct | 66 |
| Cλ156-178 | ttggagggtktggtggtctccac | 67 |
| Cλ129-148 | ttgacggggctgcyatctgc | 68 |
| Cλ93-113 | cacrgctcccgggtagaagtc | 69 |

### 1.4 Analysis of Ig V Sequence

A commercially available purification kit (QIAquick PCR purification kit; brand: Qiagen) is used to purify PCR products of VH, Vκ, and Vλ chains from the second round of PCR, and corresponding primers (SEQ ID No. 64, 66, and 69) are used for sequencing.

Then, a sequence analysis is performed by using IMGT^{®}, the international ImMunoGeneTics information system (https://www.imgt.org), so as to identify germline V(D)J gene segments having the highest sequence identity.

### 1.5 Construction of IgG Expression Vector

After sequencing, gene-specific primers (as shown in Table 3 and Table 4) are selected according to the V or J gene segment having the highest sequence identity, and the PCR products of VH, Vκ, and Vλ chains from the second round of PCR are used as a template for a further PCR. The obtained PCR products are purified and cloned into human IgG1λ expression vectors.

Then, a commercially available GeneArt^{®} seamless cloning and assembly enzyme mix (brand: Invitrogen) is used for recombination. 5 µl of a recombinant product and an *E. coli* competent cell are mixed and undergo gene transfer at 42°C. Screening of gene-transferred *E. coli* colonies is performed by using pIgG1κ-screen+ (SEQ ID No. 109) or pIgG1λ-screen+ (SEQ ID No.110) as shown in Table 5 as a forward primer and using pIgG1-screen- (SEQ ID No.111) as a reverse primer.

After PCR products of an expected size (about 1,800 bps) are sequenced to confirm identity with the original PCR products, the screened colonies are cultured at 37°C for 16 hours in a Luria-Bertani broth that contains 100 µg/ml of ampicillin. Afterwards, a commercially-available QIAprep^{®} spin columns (brand: Qiagen) is used to isolate a plasmid DNA from 3 ml of an *E. coli* culture.

**Table 3**

| Single human B Cell | V_{H} chain | V_{L} chain |
|---|---|---|
| | Forward primer / Reverse primer (SEQ ID No.) | |
| Cell-1B 10 | 76/80 | 85/92 |
| Cell-2B8 | 76/80 | 86/90 |
| Cell-1E6 | 76/82 | 84/90 |
| Cell-1E9 | 76/80 | 97/105 |
| Cell-1E12 | 76/80 | 88/90 |
| Cell-1B7 | 75/80 | 97/105 |
| Cell-1D1 | 77/83 | 84/90 |
| Cell-1G5 | 76/80 | 84/90 |
| Cell-2F1 | 72/80 | 84/90 |
| Cell-2F10 | 70/83 | 86/93 |
| Cell-2G5 | 70/80 | 84/90 |
| Cell-2G7 | 70/83 | 86/93 |
| Cell-2G11 | 70/82 | 86/90 |

**Table 4**

| Primer | 5'-3' Sequence | SEQ ID No. |
|---|---|---|
| SL-VH1/3/5f | gttgctacgcgtgtcctgagcsaggtgcagctggtgsagtc | 70 |
| SL-VH1-3f | gttgctacgcgtgtcctgagccaggtccagcttgtgcagtc | 71 |
| SL-VH1-18f | gttgctacgcgtgtcctgagccaggttcagctggtgcagtc | 72 |
| SL-VH1-24f | gttgctacgcgtgtcctgagccaggtccagctggtacagtctg | 73 |
| SL-VH2f | gttgctacgcgtgtcctgagccaggtcaccttgarggagtctg | 74 |
| SL-VH3-23f | gttgctacgcgtgtcctgagcgaggtgcagctgttggagtct | 75 |
| SL-VH4f | gttgctacgcgtgtcctgagccagstgcagctgcaggagt | 76 |
| SL-VH4-34f | gttgctacgcgtgtcctgagccaggtgcagctacarcagtgg | 77 |
| SL-VH6f | gttgctacgcgtgtcctgagccaggtacagctgcagcagtca | 78 |
| SL-VH7f | gttgctacgcgtgtcctgagccaggtgcagctggtgcaat | 79 |
| SL-JH1/4/5r | gatgggcccttggtgctagctgaggagacggtgaccagg | 80 |
| SL-JH2r | gatgggcccttggtgctagctgaggagacagtgaccagggt | 81 |
| SL-JH3r | gatgggcccttggtgctagctgaagagacggtgaccattgtc | 82 |
| SL-JH6r | gatgggcccttggtgctagctgaggagacggtgaccgtg | 83 |
| SL-VK1f | ggctcccaggtgcacgatgtgacatccagwtgacccagtctcc | 84 |
| SL-VK2f | ggctcccaggtgcacgatgtgatattgtgatgacccagactccactc t | 85 |
| SL-VK3f | ggctcccaggtgcacgatgtgaaattgtgttgacrcagtctccag | 86 |
| SL-VK4f | ggctcccaggtgcacgatgtgacatcgtgatgacccagtctc | 87 |
| SL-VK5f | ggctcccaggtgcacgatgtgaaacgacactcacgcagtctc | 88 |
| SL-VK6f | ggctcccaggtgcacgatgtgaaattgtgctgactcagtctcca | 89 |
| SL-JK1r | tgcagccaccgtacgtttgatttccaccttggtccct | 90 |
| SL-JK2r | tgcagccaccgtacgtttgatctccagcttggtccct | 91 |
| SL-JK3r | tgcagccaccgtacgtttgatatccactttggtccca | 92 |
| SL-JK4r | tgcagccaccgtacgtttgatctccaccttggtccct | 93 |
| SL-JK5r | tgcagccaccgtacgtttaatctccagtcgtgtccctt | 94 |
| SL-LV1-40/50/51f | tccttgcttatgggtccggagtggattctcagtctgtgytgacgcagc c | 95 |
| SL-LV1-36/44/47f | tccttgcttatgggtccggagtggattctcagtctgtgctgactcagcc a | 96 |
| SL-LV2f | tccttgcttatgggtccggagtggattctcagtctgccctgactcagc c | 97 |
| SL-LV3f | tccttgcttatgggtccggagtggattcttcctatgagctgacwcagc ca | 98 |
| SL-LV3-19f | tccttgcttatgggtccggagtggattcttcttctgagctgactcagga c | 99 |
| SL-LV4/5/9f | tccttgcttatgggtccggagtggattctcagsctgtgctgactcagc c | 100 |
| SL-LV4-60/69f | tccttgcttatgggtccggagtggattctcagyctgtgctgactcaatc | 101 |
| SL-LV6f | tccttgcttatgggtccggagtggattctaattttatgctgactcagccc | 102 |
| SL-LV7/8f | tccttgcttatgggtccggagtggattctcagrctgtggtgacycagg ag | 103 |
| SL-LV10f | tccttgcttatgggtccggagtggattctcaggcagggctgactcag cc | 104 |
| SL-JL1r | ggccttgggctgacctaggacggtgaccttggtcc | 105 |
| SL-JL2/3f | ggccttgggctgacctaggacggtcagcttggtcc | 106 |
| SL-JL6r | ggccttgggctgacctaggacggtcaccttggtgc | 107 |
| SL-JL7r | ggccttgggctgacctaggacggtcagctgggtgc | 108 |

**Table 5**

| Primer | 5'-3' Sequence | SEQ ID No. |
|---|---|---|
| pIgG₁κ-screen+ | gctcccaggtgcacgatgtg | 109 |
| pIgG₁λ-screen+ | gcttatgggtccggagtggattct | 110 |
| pIgG₁-screen- | gatgggcccttggtgctagc | 111 |

### 1.6 Production and Purification of Recombinant Antibody

A commercially available medium (a FreeStyle^{™} 293 expression medium; brand: Gibco) is used to culture FreeStyle^{™} 293-F cells (brand: Thermo Scientific) in a 250 ml culture flask at a concentration of 1 x 10⁶ cells. Linear polyethylenimine (PEI) having an average molecular weight of 25 kDa (brand: Polysciences) is used as a transfection reagent. 88 µg of the above-mentioned plasmid DNA and the exponentially growing FreeStyle^{™} 293-F cells (1.5 to 2 x 10⁶ cells) are transfected and cultured for 3 days. The medium is harvested and centrifuged at 3,000 rpm for 10 minutes. Then, FreeStyle^{™} 293-F cell debris is removed, and a 0.45 µm filter is used to filter the resultant supernatant.

The filtered supernatant is further purified with a commercially available protein purification product (Protein A Sepharose Fast Flow beads; brand: GE Healthcare), so as to obtain a recombinant antibody. Specifically, 80 ml of said filtered supernatant is added into 80 µl of the Protein A Sepharose Fast Flow beads, and a mixture thereof is aliquoted into two 50 ml tubes for 24-hour rotation and incubation at 4°C. Then, the tubes were centrifuged at 3,000 rpm for 10 minutes, the supernatant is removed, and washing is performed with PBS. Elution is further performed with 0.1 M of glycine (pH 3.0), and eluates ate collected in tubes containing 1 M of Tris (pH 8.0). By using the PBS for dialysis, thirteen anti-PLA2R monoclonal antibodies can be obtained, which are: an anti-PLA2R monoclonal antibody 1B10 (hereunder referred to as 1B10 or antibody 1B10), an anti-PLA2R monoclonal antibody 2B8 (hereunder referred to as 2B8 or antibody 2B8), an anti-PLA2R monoclonal antibody 1E6 (hereunder referred to as 1E6 or antibody 1E6), an anti-PLA2R monoclonal antibody 1E9 (hereunder referred to as 1E9 or antibody 1E9). an anti-PLA2R monoclonal antibody 1E12 (hereunder referred to as 1E12 or antibody 1E12), an anti-PLA2R monoclonal antibody 1B7 (hereunder referred to as 1B7 or antibody 1B7), an anti-PLA2R monoclonal antibody 1D1 (hereunder referred to as 1D1 or antibody 1D1), an anti-PLA2R monoclonal antibody 1G5 (hereunder referred to as 1G5 or antibody 1G5), an anti-PLA2R monoclonal antibody 2F1 (hereunder referred to as 2F1 or antibody 2F1), an anti-PLA2R monoclonal antibody 2F10 (hereunder referred to as 2F10 or antibody 2F10), an anti-PLA2R monoclonal antibody 2G5 (hereunder referred to as 2G5 or antibody 2G5), an anti-PLA2R monoclonal antibody 2G7 (hereunder referred to as 2G7 or antibody 2G7), and an anti-PLA2R monoclonal antibody 2G11 (hereunder referred to as 2G11 or antibody 2G11).

### [Embodiment 2 Construction of Truncated sPLA2R and PLA2R Proteins]

### 2.1 Construction of sPLA2R

Based on a method published by Kanigicherla D, et al. (2013), a commercially available pSectag2B vector can be used to clone an extracellular DNA sequence (gene ID: 22925) of a wild-type human PLA2R into two parts, in which part A covers nucleotide sequences of 60-1788 and part B covers nucleotide sequences of 1545-4191. After sequence accuracy is verified by EcoRV and XhoI, parts A and B are ligated and recloned into the pSectag2B vector, so as to obtain a plasmid expressing a full-length PLA2R protein gene.

After introducing pSectag2B-sPLA2R into FreeStyle 293 cells, and culturing said cells in a serum-free FreeStyle 293 medium for three days, the medium is condensed, and affinity chromatography is used to purify a recombinant PLA2R protein in a soluble form (hereunder referred to as sPLA2R).

### 2.2 Construction of Truncated PLA2R Protein

By using the cDNA (gene ID: 22925) of the wild-type human PLA2R as a template, and using the above-mentioned pSectag2B vector and expression system, a truncated PLA2R protein without amino acid residues can be manufactured (as shown in Table 6).

**Table 6**

| Truncated PLA2R protein | |
|---|---|
| ΔF | deletion of amino acid residues from Gln-36 to Thr-223 |
| Δ1 | deletion of amino acid residues from Gln-36 to Tyr-357 |
| Δ2 | deletion of amino acid residues from Gln-36 to Ala-504 |
| Δ3 | deletion of amino acid residues from Gln-36 to Pro-660 |
| Δ4 | deletion of amino acid residues from Gln-36 to Lys-805 |
| Δ5 | deletion of amino acid residues from Gln-36 to Lys-947 |
| Δ6 | deletion of amino acid residues from Gln-36 to His-1105 |
| Δ7 | deletion of amino acid residues from Gln-36 to Pro-1236 |
| CyRs | deletion of amino acid residues from Gln-36 to Asp-165 |
| CTLD1 | deletion of amino acid residues from Gln-36 to Tyr-357 |

### [Embodiment 3 Analysis and Activity Evaluation of Anti-PLA2R Monoclonal Antibodies]

### 3.1 Sequence Analysis of Anti-PLA2R Monoclonal Antibodies

Based on the IMGT^{®} (Lefranc MP, et al. Nucleic Acids Res. 1999;27(1):209-212.), the sequence analysis is performed on the anti-PLA2R monoclonal antibodies by using sequence annotations and a network-based sequence analysis (https://www.imgt.org/IMGT_vquest/share/textes/index.html and https://www.ncbi.nlm.nih.gov/igblast/).

The CDRs of the anti-PLA2R monoclonal antibodies are shown in Table 7 and Table 8, and genetics analysis results are shown in Table 9.

The anti-PLA2R monoclonal antibodies are primarily of the IgG4 subclass. However, four of the antibodies are of the IgG1 subclass, two of the antibodies are of the IgG3 subclass, and two of the antibodies are of the IgG2 subclass, all of which are predominantly κ light chains. Furthermore, despite exhibiting variations in somatic hypermutation, the antibody 2G7 and the antibody 2F10 sharing the IGHV1-69-D2-15-J6 and IGKV3-20-J4 coding sequences are recognized to be of the same clonal type from one single source. Ten of the thirteen antibodies have the J1-1 gene, but the dominant V gene is not detectable. All thirteen antibodies have somatic mutations, and the IgH V, Igκ V, and Igλ V genes have an average of 14.4 ± 2.3, 9.7 ± 3.2, and 13.5 ± 1.5 mutations, respectively. Compared with healthy control groups (based on past reported research data), average mutation rates of the IgH V and Igκ V genes are similar, and a mutation rate of the Igλ V gene is two times more. This indicates involvement of T cell-dependent B cell maturation.

**Table 7**

| Antibody | H-CDR1 | H-CDR2 | H-CDR3 |
|---|---|---|---|
| 1B10 | GGSISSFS (SEQ ID No:112) | IYYSGST (SEQ ID No:113) | GKGPRSNSPLGV (SEQ ID No:114) |
| 2B8 | GGSFTRYY (SEQ ID No:115) | ISHNGGP (SEQ ID No:116) | ARETGGDYFDI (SEQ ID No:117) |
| 1E6 | GGPINNYF (SEQ ID No:118) | IYISGTT (SEQ ID No:119) | ARGADTFDV (SEQ ID No:120) |
| 1E9 | GGSINSFS (SEQ ID No:121) | IYYSGST (SEQ ID No:122) | GRMLRGRTDS (SEQ ID No:123) |
| 1E12 | GGSISSYS (SEQ ID No:124) | VYYSGNT (SEQ ID No:125) | VRGAQQRMLAY (SEQ ID No:126) |
| 1B7 | GFTFSTYD (SEQ ID No:127) | VNGRGDKT (SEQ ID No:128) | VMETPSSAWSGD (SEQ ID No:129) |
| 1D1 | GGSFSDYY (SEQ ID No:130) | INHSGST (SEQ ID No:131) | ARGRLRGELPAA GPPHAHKPAMYM DV (SEQ ID No:132) |
| 1G5 | GGSISNSGHY (SEQ ID No:133) | TSSSGYT (SEQ ID No:134) | ARLKNIWGAPGA FDY (SEQ ID No:135) |
| 2F1 | GYTFTTYA (SEQ ID No: 136) | ISPYKVNT (SEQ ID No:137) | ARLQSPPGSFASG SYYFNP (SEQ ID No:138) |
| 2F10 | GGTFRSYT (SEQ ID No:139) | IIPVFGTT (SEQ ID No:140) | ARDHSTYYYMD V (SEQ ID No:141) |
| 2G5 | GGTFSSSA (SEQ ID No:142) | IVPIFRTA (SEQ ID No:143) | AGRSGSYRGAFH D (SEQ ID No:144) |
| 2G7 | GGNFRSYT (SEQ ID No:145) | IIPVFGTT (SEQ ID No:146) | ARDHSTYYYMD V (SEQ ID No:147) |
| 2G11 | GFSFSSYW (SEQ ID No:148) | IYPGDSET (SEQ ID No:149) | ARFSGIERITLSGD ALDM (SEQ ID No:150) |

**Table 8**

| Antibody | L-CDR1 | L-CDR2 | L-CDR3 |
|---|---|---|---|
| 1B10 | QSLLHTNEYNY (SEQ ID No:151) | LGS (SEQ ID No:152) | MQGLQSPFT (SEQ ID No:153) |
| 2B8 | QYISKY (SEQ ID No:154) | AAS (SEQ ID No:155) | QQSYNTPRT (SEQ ID No:156) |
| 1E6 | QDIRNY (SEQ ID No:157) | AAS (SEQ ID No:158) | QHYYTYPRA (SEQ ID No:159) |
| 1E9 | NSDVGAYKF (SEQ ID No:160) | DVS (SEQ ID No:161) | NSYADSYFYV (SEQ ID No:162) |
| 1E12 | QSISSW (SEQ ID No:163) | KAS (SEQ ID No:164) | QQYNSYPWT (SEQ ID No:165) |
| 1B7 | NSDVGAYKF (SEQ ID No:166) | DVS (SEQ ID No:167) | NSYADSYFYV (SEQ ID No:168) |
| 1D1 | QTIIT* (SEQ ID No:169) | AAS (SEQ ID No:170) | QQTHTTPVT (SEQ ID No: 171) |
| 1G5 | QDIGNY (SEQ ID No:172) | DAS (SEQ ID No:173) | QQYGDLWT (SEQ ID No:174) |
| 2F1 | QSIGRY (SEQ ID No:175) | AAS (SEQ ID No:176) | QQSFNSPPT (SEQ ID No:177) |
| 2F10 | QSVDNRY (SEQ ID No:178) | DAS (SEQ ID No:179) | QQFASTLT (SEQ ID No:180) |
| 2G5 | QNVSTW (SEQ ID No:181) | KAS (SEQ ID No:182) | QQYKSYWT (SEQ ID No:183) |
| 2G7 | ESVDTRY (SEQ ID No:184) | DAS (SEQ ID No:185) | QQFASTLT (SEQ ID No:186) |
| 2G11 | QSLSSSY (SEQ ID No:187) | DAS (SEQ ID No:188) | HQYLSSLWT (SEQ ID No:189) |

**Table 9**

| **Patient** | **mAb** | **Isotype** | | **Heavy chain VDJ genes** | | | **VH mutations nt (%)** | **Light chain VJ genes** | | **VL mutations nt (%)** |
|---|---|---|---|---|---|---|---|---|---|---|
| 1 | 2B8 | IgG3 | k | V4-59*01 | D2-8*02 | J4* 02 | 24/293 (8%) | V1-39*01 | J1*01 | 9/286 (3%) |
| 2 | 1E6 | IgG1 | k | V4-4*07 | D3-10*01 | J3* 01 | 29/289 (10%) | V1-8*01 | J1*01 | 22/286 (8%) |
| 2 | 1E9 | IgG2 | l | V4-59*01 | D3-10*01 | J4* 02 | 23/286 (8%) | V2-11*01 | J1*01 | 26/295 (9%) |
| 2 | 1E12 | IgG3 | k | V4-59*01 | D6-25*01 | J4* 02 | 18/292 (6%) | V1-5*03 | J1*01 | 5/281 (2%) |
| 3 | 1B7 | IgG2 | l | V3-23*01 | D6-13*01 | J5* 02 | 21/292 (7%) | V2-11*01 | J1*01 | 18/295 (6%) |
| 3 | 1B10 | IgG1 | k | V4-59*01 | D2-2*01 | J6* 02 | 24/292 (8%) | V2-28*01 | J3*01 | 12/300 (4%) |
| 4 | 2F1 | IgG1 | k | V1-18*01 | D3-10*01 | J4* 02 | 28/294 (10%) | V1-39*01 | J1*01 | 22/287 (8%) |
| 4 | 2G7 | IgG4 | k | V1-69*01 | D2-15*01 | J6* 03 | 25/296 (8%) | V3-20*01 | J4*01 | 17/282 (6%) |
| 4 | 2F10 | IgG4 | k | V1-69*12 | D2-15*01 | J6* 03 | 24/296 (8%) | V3-20*01 | J4*01 | 13/286 (5%) |
| 4 | 2G11 | IgG4 | k | V5-51*01 | D5-24*01 | J3* 02 | 26/294 (9%) | V3-20*01 | J1*01 | 22/286 (8%) |
| 4 | 1G5 | IgG1 | k | V4-39*07 | D1-26*01 | J4* 02 | 30/297 (10%) | V1-33*01 | J1*01 | 14/281 (5%) |
| 4 | 2G5 | IgG4 | k | V1-69*06 | D1-26*01 | J1* 01 | 28/291 (10%) | V1-5*01 | J5*01 | 17/283 (6%) |

### 3.2 Specificity Analysis of Anti-PLA2R Monoclonal Antibodies

In order to determine whether or not the anti-PLA2R monoclonal antibody can bind to the PLA2R protein, a phospholipase A2 receptor (PLA2R) is used in cooperation with the western blot. In addition, the purified sPLA2R (soluble PLA2R) is used in cooperation with an enzyme-linked immunosorbent assay (ELISA) for analyzing a binding capacity of the anti-PLA2R monoclonal antibody to the PLA2R.

Cell fluorescence staining is adopted for analyzing specificity of the anti-PLA2R monoclonal antibody. Specifically, HEK293 cells overexpressing mPLA2R-EGFP (membrance PLA2R conjugated GFP) can be constructed by use of a conventional technique, and podocytes overexpressing mPLA2R-EGFP can be constructed via a lentiviral system. Then, immunofluorescence is performed on the HEK293 cells overexpressing mPLA2R-EGFP and the podocytes overexpressing mPLA2R-EGFP by using the anti-PLA2R monoclonal antibody (primary) in cooperation with an Alexa 594-conjugated mouse anti-human IgG secondary antibody (brand: Jackson Immuno Research) and a nucleus dye (Hoechst 33342; brand: Invitrogen).

The specificity of the anti-PLA2R monoclonal antibody is further analyzed by use of a flow cytometer. Specifically, 1 x 10⁶ podocytes expressing mPLA2R and the anti-PLA2R monoclonal antibody (1 µg/ml) are incubated on ice for one hour. After incubation, an APC anti-human IgG Fc antibody (brand: BioLegend) is added for another 30-minute incubation on ice, and the flow cytometer (brand: BD FACSVerse) is used to detect a fluorescent signal.

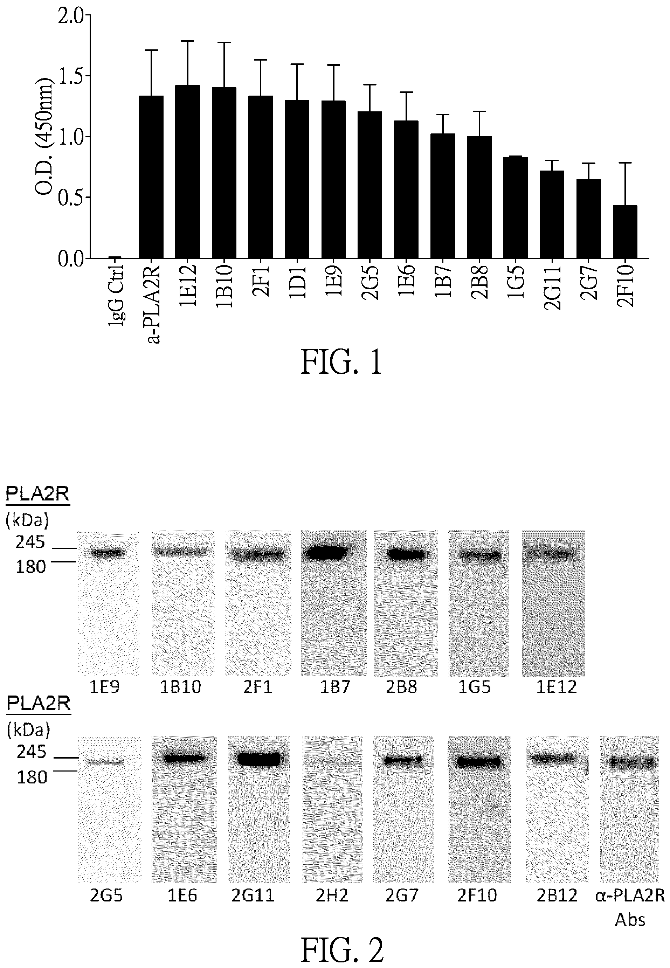

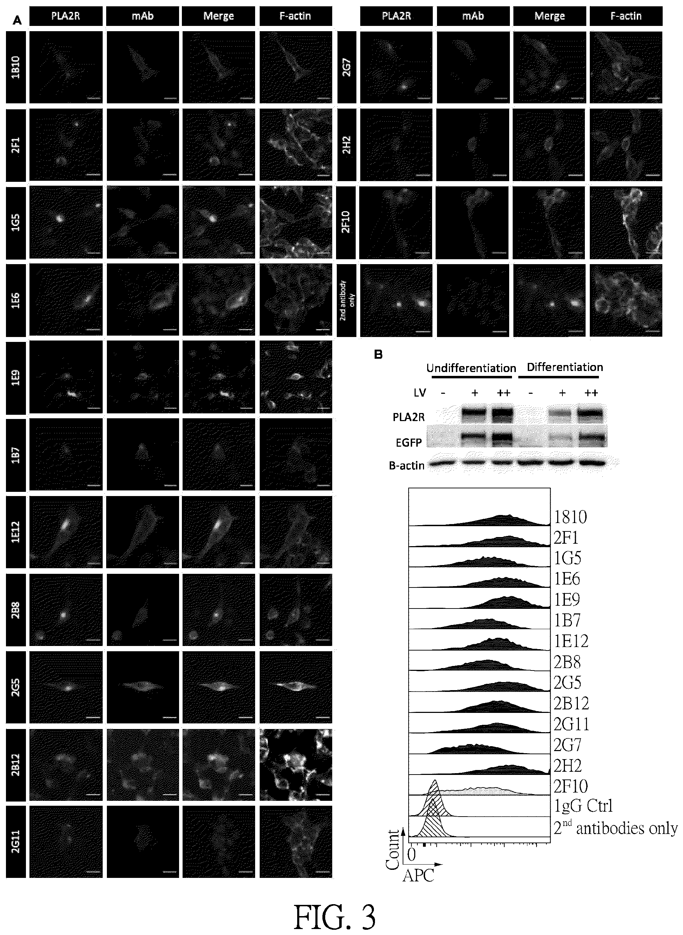

An ELISA analysis result and a western blot analysis result are shown in FIG.1 and FIG. 2. All of the anti-PLA2R monoclonal antibodies can bind to the sPLA2R. Analysis results of the cell fluorescence staining and the flow cytometer are shown in FIG. 3. In the results of the cell fluorescence staining, combination of anti-PLA2R monoclonal antibody signals (red fluorescence) and mPLA2R-EGFP signals (green fluorescence) can be observed in all of the anti-PLA2R monoclonal antibodies. Such results indicate that all of the anti-PLA2R monoclonal antibodies can recognize mPLA2R. Furthermore, the analysis results of the flow cytometer indicate that all of the anti-PLA2R monoclonal antibodies can bind to the podocytes expressing mPLA2R.

It can be observed from the analysis results of FIG. 1 to FIG. 3 that, despite the anti-PLA2R monoclonal antibodies exhibiting different levels of binding affinity, all of the anti-PLA2R monoclonal antibodies can bind to the sPLA2R and the mPLA2R (regardless of subclasses of the anti-PLA2R monoclonal antibodies).

### 3.3 Analysis of Binding Epitopes of Anti-PLA2R Monoclonal Antibodies

With use of the truncated PLA2R protein manufactured above, the western blot is adopted to analyze binding epitopes of the anti-PLA2R monoclonal antibodies.

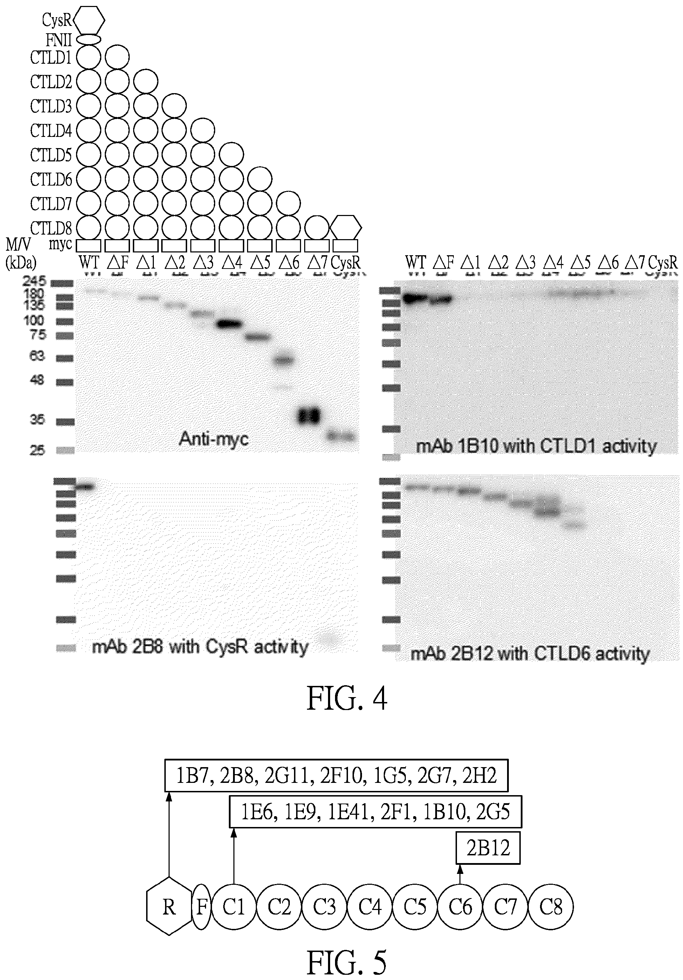

Results are shown in FIG. 4 and FIG. 5. The anti-PLA2R monoclonal antibodies can identify three different PLA2R epitopes (CysR, CTLD1, and CTLD6). While six of the anti-PLA2R monoclonal antibodies (1B7, 2B8, 2G11, 2F10, 1G5, 2G7, and 2H2) bind to the PLA2R via CysR, six of the anti-PLA2R monoclonal antibodies (1E6, 1E9, 1E12, 2F1, 1B10, and 2G5) bind to the PLA2R via CTLD1. Such results indicate that the main binding sites of the anti-PLA2R monoclonal antibodies are a CysR domain and a CTLD1 domain at a protein N-terminus of the PLA2R.

### 3.4 Affinity Analysis of Anti-PLA2R Antibodies and PLA2R Protein

Bio-layer interferometry (BLI) is adopted to perform an antibody affinity analysis, which includes using the ForteBio's Octet RED96 system to measure an association rate constant (Kₐ) and a dissociation rate constant (K_{d}).

Specifically, an anti-human IgG Fc capture (AHC) biosensor (brand: ForteBio) is used to capture the anti-PLA2R monoclonal antibodies having a concentration of 750 ng/ml, and to achieve a 0.5 nm shift. Then, said biosensor is immersed into sPLA2R solutions having different concentrations (0 nM, 3.125 nM, 6.25 nM, 12.5 nM, 25 nM, 50 nM, 100 nM, and 125 nM). A running buffer is sterile water containing 0.1% of bovine serum albumin (BSA), 0.1% of Tween 20, 250 mM sodium chloride (NaCl), 2.7 mM potassium chloride (KCl), 10 mM disodium phosphate (Na₂HPO₄), and 1.8 mM monopotassium phosphate (KH₂PO₄). Through binding reactions occurred during a 5-minute association phase and a 15-minute dissociation phase, a curve fitting analysis (with use of a 1:1 Langmuir binding model) is performed to determine a reaction rate constant.

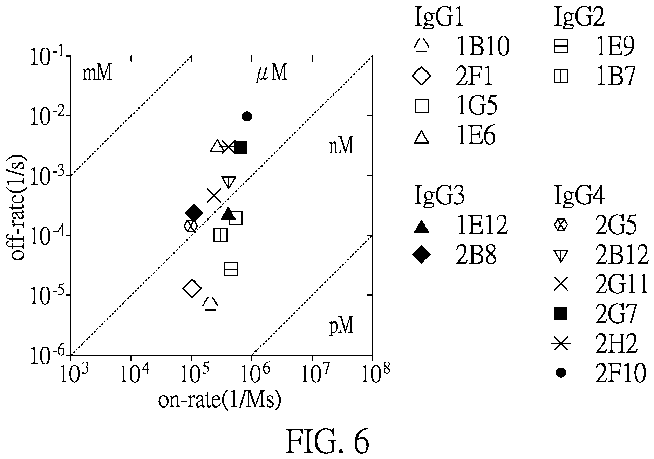

Results of the antibody affinity analysis are shown in Table 10 and FIG. 6. For the thirteen anti-PLA2R monoclonal antibodies, their equilibrium dissociation constants (K_{D} = K_{d}/Kₐ) fall within a range of between 1.16 × 10⁻⁸ M and 3 × 10⁻¹¹ M, their association rate constants (Kₐ) fall within a range of between 8.3 × 10⁴ 1/Ms and 8.6 × 10⁵ 1/Ms, and their dissociation rate constants (K_{d}) fall within a range of between 7.18 × 10⁻⁶ 1/s and 9.93 × 10⁻³ 1/s. In addition, the anti-PLA2R monoclonal antibodies designated as 1B10, 2B8, 1E6, 1E9, 1E12, 1B7, 1D1, 1G5, 2F1, 2F10, 2G5, 2G7, and 2G11 have low K_{D} values, which indicates good affinity between these anti-PLA2R monoclonal antibodies and the PLA2R protein.

It should be noted that the equilibrium dissociation constants (K_{D}) of the anti-PLA2R monoclonal antibodies of the IgG1 subclass are mostly less than 10⁻⁹ M, and the equilibrium dissociation constants (K_{D}) of the anti-PLA2R monoclonal antibodies of the IgG4 subclass are all greater than 10⁻⁹ M. Such results indicate that the anti-PLA2R monoclonal antibodies of the IgG1 subclass have higher affinity for the PLA2R protein as compared with the anti-PLA2R monoclonal antibodies of the IgG4 subclass.

**Table 10**

| mAb | Kₐ (1/Ms) | K_{d} (1/s) | K_{D} (M) |
|---|---|---|---|
| 1B10 | 2.04 x 10⁵ | 7.18 x 10⁻⁶ | 3.00 x 10⁻¹¹ |
| 2B8 | 1.14 x 10⁵ | 2.36 x 10⁻⁴ | 2.08 x 10⁻⁹ |
| 1E6 | 2.68 x 10⁵ | 3.30 x 10⁻³ | 1.23 x 10⁻⁸ |
| 1E9 | 4.79 x 10⁵ | 2.89 x 10⁻⁵ | 6.04 x 10⁻¹¹ |
| 1E12 | 4.09 x 10⁵ | 2.50 x 10⁻⁴ | 6.13 x 10⁻¹⁰ |
| 1B7 | 3.05 x 10⁵ | 1.05 x 10⁻⁴ | 3.43 x 10⁻¹⁰ |
| 1D1 | 8.30 x 10⁴ | 5.44 x 10⁻⁵ | 6.56 x 10⁻¹⁰ |
| 1G5 | 5.32 x 10⁵ | 2.04 x 10⁻⁴ | 3.83 x 10⁻¹⁰ |
| 2F1 | 1.03 x 10⁵ | 1.36 x 10⁻⁵ | 1.33 x 10⁻¹⁰ |
| 2F10 | 8.60 x 10⁵ | 9.96 x 10⁻³ | 1.16 × 10⁻⁸ |
| 2G5 | 9.71 x 10⁴ | 1.50 x 10⁻⁴ | 1.55 x 10⁻⁹ |
| 2G7 | 6.39 x 10⁵ | 3.02 x 10⁻³ | 4.72 x 10⁻⁹ |
| 2G11 | 2.34 x 10⁵ | 4.78 x 10⁻⁴ | 2.04 10⁻⁹ |

### 3.5 Analysis of Antigenic Epitope Binning

The bio-layer interferometry (BLI) is adopted to perform an antigenic epitope binning analysis of the anti-PLA2R monoclonal antibodies.

Firstly, a first one of the anti-PLA2R monoclonal antibodies is loaded onto the AHC biosensor, so as to obtain a fixed response. After 100-second and 120-second baseline steps, the sPLA2R is added in a dose-dependent manner during a 600-second association step. Then, a second one of the anti-PLA2R monoclonal antibodies is loaded onto the AHC biosensor for 600-second incubation. If signal accumulation can be observed, it is indicated that binding of each of these two antibodies occurs at a different epitope. If no signal accumulation is observed, it is indicated that binding of each of these two antibodies occurs at the same epitope.

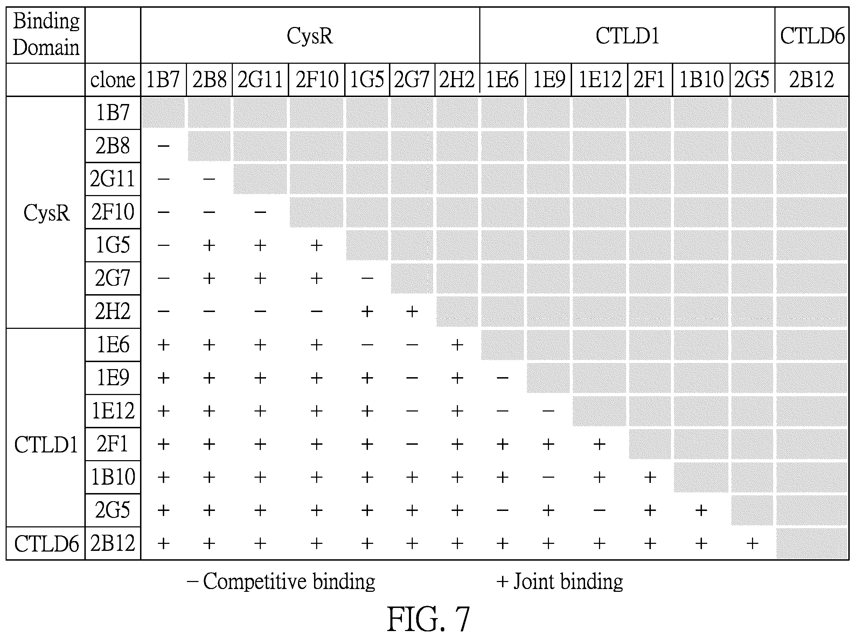

Results of a competitive analysis on antigenic epitopes between the anti-PLA2R monoclonal antibodies are shown in FIG. 7. Cross competition exists between the anti-PLA2R monoclonal antibodies that show reactivity to CysR and CTLD1. It should be noted that binding interference between CysR and CTLD1 exists in the antibody 2G7 and the antibody 1G5 that show reactivity to CysR, and in the antibody 1E6 that shows reactivity to CTLD1.

Specifically, the binding interference is generated between the antibody 2G7 and most (four out of six) of the anti-PLA2R monoclonal antibodies targeting CTLD1. This indicates that the sPLA2R binding site of the antibody 2G7 may span from CysR to CTLD1. Moreover, compared with the antibody 2G7, the antibody 1G5, and the antibody 1E6, other eleven anti-PLA2R monoclonal antibodies exhibit restricted binding epitopes, only a few of which interfere with binding of the anti-PLA2R monoclonal antibodies that are recognized to have the same domain.

### 3.6 Activity Analysis of Anti-PLA2R Monoclonal Antibodies of Different IgG Subclasses for Inducing CDC

A commercially available MTT assay reagent (CellTiter-Glo luminescent cell viability assay; brand: Promega) is used to analyze activity of the anti-PLA2R monoclonal antibodies of different IgG subclasses for inducing complement-dependent cytotoxicity (CDC).

Specifically, after original podocytes (cell line: AB8/13) or podocytes overexpressing PLA2R (cell line: PLA2R-AB8/13; hereunder referred to as PLA2R-podocytes) that suspend in an RPMI 1640 medium containing 10% of FBS are seeded and cultured overnight in a 96-well flat-bottomed white plate (5 x 10³ cells/well), said medium is replaced by an RPMI medium containing 2% of FBS (100 µl per well), and the anti-PLA2R monoclonal antibody (6.6 nM) is added for incubation at 37°C for 1 hour. Then, 5% of baby rabbit complement (brand: Cedarlane) is added into each well for incubation at 37°C for 4 hours. After 100 µL of a CellTiter-Glo reagent is further added and incubated with shaking at 70 rpm for 15 minutes at a room temperature, a plate reader (brand: PerkinElmer) is used for measurement of a luminescence value.

In order to determine how the activity of the anti-PLA2R monoclonal antibody for inducing the CDC is affected by the IgG subclass, antibody engineering is employed for changing the IgG subclass of the anti-PLA2R monoclonal antibody. For example, the Fc fragment of the anti-PLA2R monoclonal antibody of the IgG1 subclass is replaced by the Fc fragment of the IgG4 subclass, so as to change the subclass of the anti-PLA2R monoclonal antibody from IgG1 to IgG4. Alternatively, the Fc fragment of the anti-PLA2R monoclonal antibody of the IgG4 subclass is replaced by the Fc fragment of the IgG1 subclass, so as to change the subclass of the anti-PLA2R monoclonal antibody from IgG4 to IgG1.

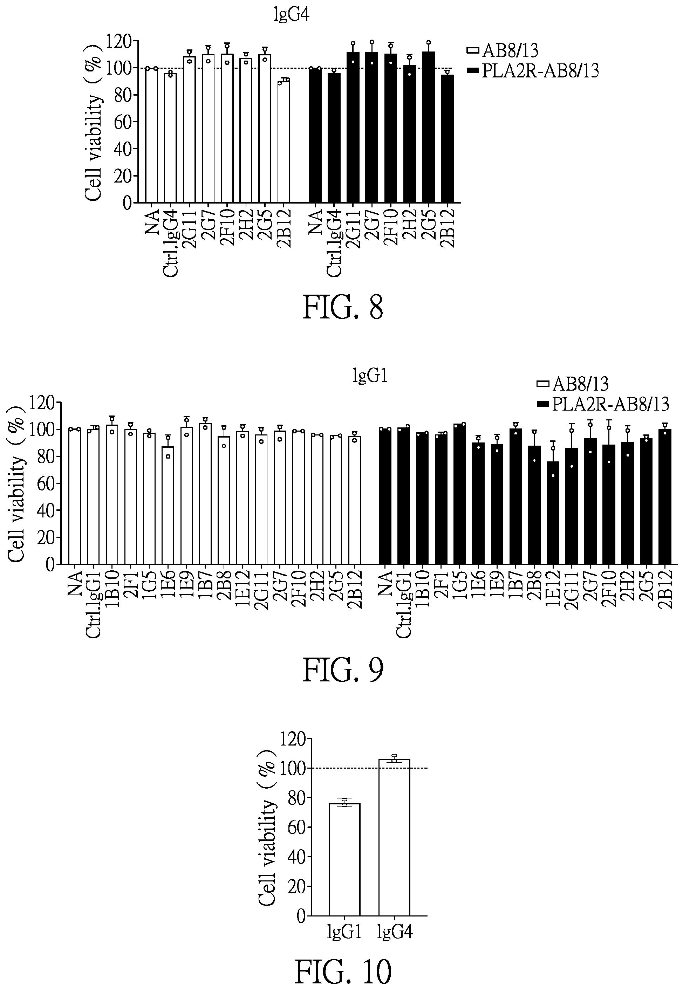

Results of the activity of the anti-PLA2R monoclonal antibodies of different subclasses for inducing the CDC are shown in FIG. 8 to FIG. 13. From the results as shown in FIG. 8, it can be observed that the anti-PLA2R monoclonal antibodies of the IgG4 subclass have no significant influence on cell viability of the original podocytes and the PLA2R-podocytes. All IgG4 antibody variants do not have the activity to induce the CDC.

The antibody 1E12 of the IgG1 subclass (which recognizes CTLD1) can induce the CDC in the PLA2R-podocytes, and the cell viability is reduced to merely 76% (as shown in FIG. 9). However, if the subclass of the antibody 1E12 is changed to IgG4, there is no significant toxicity to cells (as shown in FIG. 10). Such results indicate that the anti-PLA2R monoclonal antibodies rely on the Fc fragment of IgG1 to induce a CDC response.

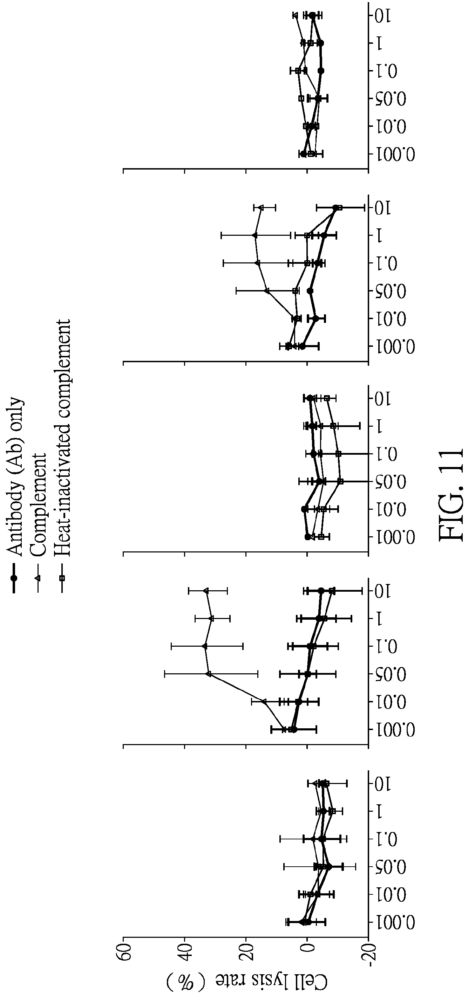

From the results as shown in FIG. 11, the antibody 1E12 can induce 33% of cell lysis at a concentration of 10 µg/ml. Different from the antibody IB10 or the antibody 2G7, the CDC response induced by the antibody 2B8 is moderate, and 17% of cell lysis can be induced at a higher concentration. It should be noted that the CDC response induced by each of the antibody 1E12 and the antibody 2B8 can be removed by heat inactivation (at 56°C for 30 minutes) of complement in advance. This shows that the cell lysis caused by such antibodies is the result of CDC induction.

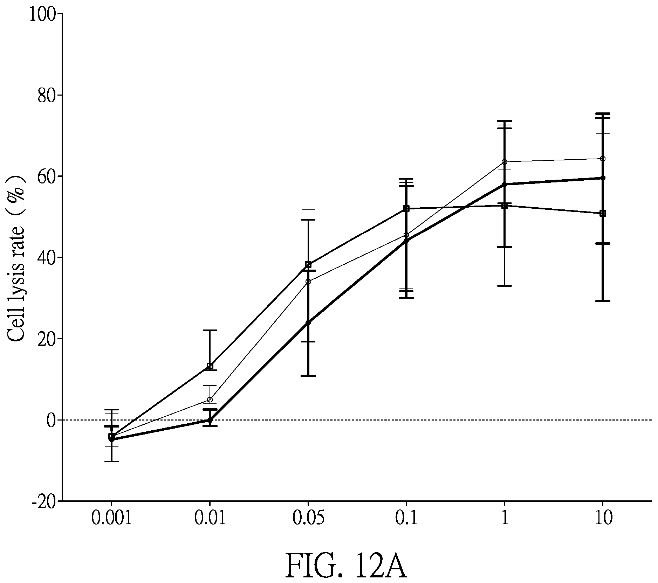

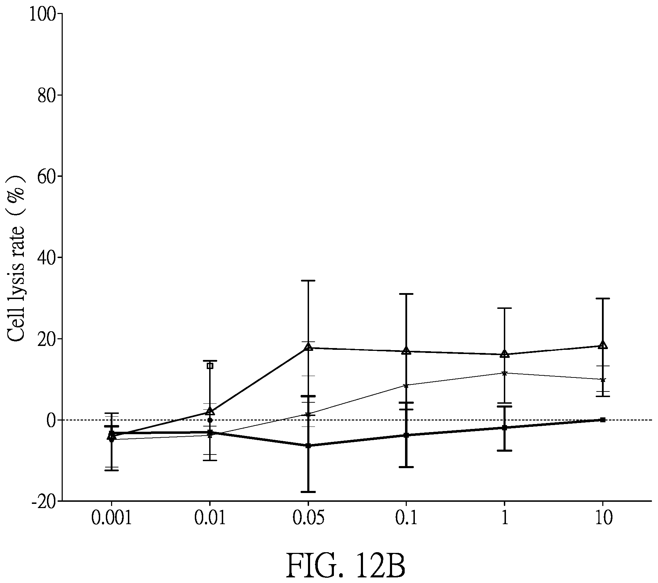

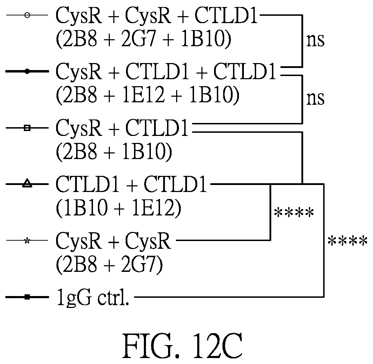

From the results as shown in FIG. 11, FIG. 12A, FIG. 12B, and FIG. 12C, it can be observed that the antibody 1B10 (which recognizes CTLD1) will not induce the CDC response in the PLA2R-podocytes. However, when the antibody 1B10 (which recognizes CTLD1) and the antibody 2B8 (which recognizes CysR) are co-existent, cytotoxicity can be significantly increased, thereby resulting in 59% of cell lysis.

In comparison, when antibodies capable of recognizing the same epitope are co-existent (e.g., the antibody 2B8 and the antibody 2G7 (both of which recognize CysR) or the antibody 1B10 and the antibody 1E12 (both of which recognize CTLD1) are co-existent), the cytotoxicity is not significantly increased, and merely 10% or 17% of cell lysis is achieved.

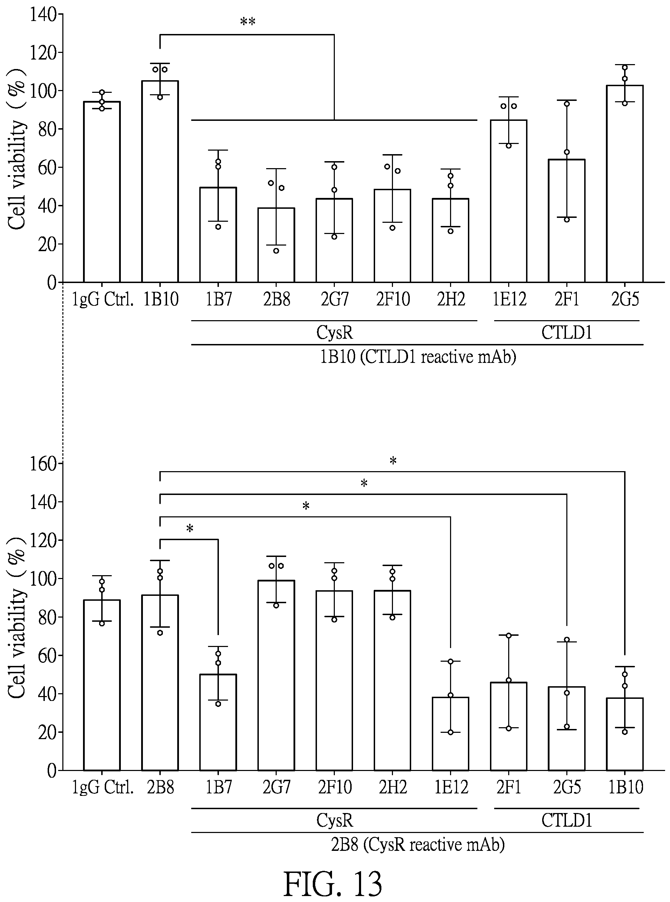

Furthermore, it can be observed from the results as shown in FIG. 13 that the activity of the antibody 1B10 (which recognizes CTLD1) for inducing the CDC response can be increased by adding the anti-PLA2R monoclonal antibody capable of recognizing CysR. Similarly, the activity of the antibody 2B8 (which recognizes CysR) for inducing the CDC response can be increased by adding the anti-PLA2R monoclonal antibody capable of recognizing CTLD1.

### 3.7 Another Activity Analysis of Anti-PLA2R Monoclonal Antibodies of Different IgG Subclasses for Inducing CDC

In order to determine how the activity of the anti-PLA2R monoclonal antibody for inducing the CDC is affected by the IgG subclass, the antibody engineering is employed for replacing the Fc fragment of the antibody 2B8 (which recognizes CysR) of the IgG3 subclass by the Fc fragment of the IgG1 subclass or the IgG4 subclass, and replacing the Fc fragment of the antibody 1B10 (which recognizes CTLD1) of the IgG1 subclass by the Fc fragment of the IgG3 subclass or the IgG4 subclass. Then, by using the commercially available MTT assay reagent in the manner as mentioned above, the activity analysis for the CDC induction is performed.

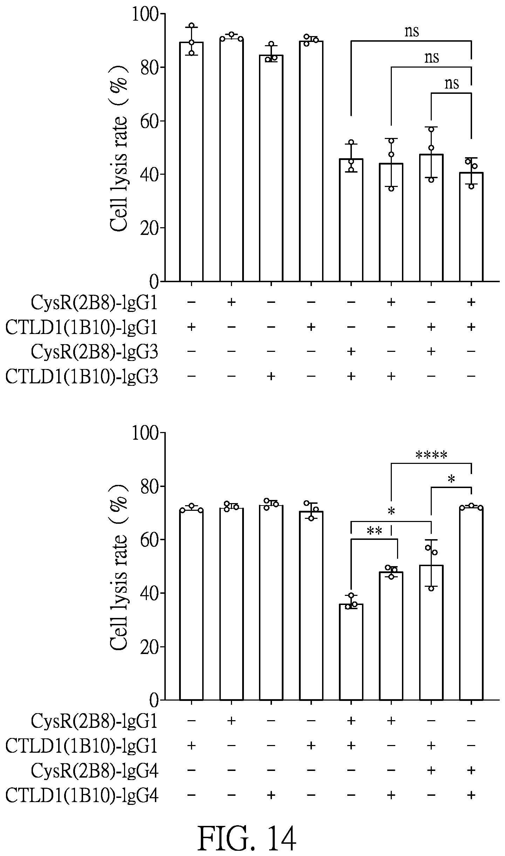

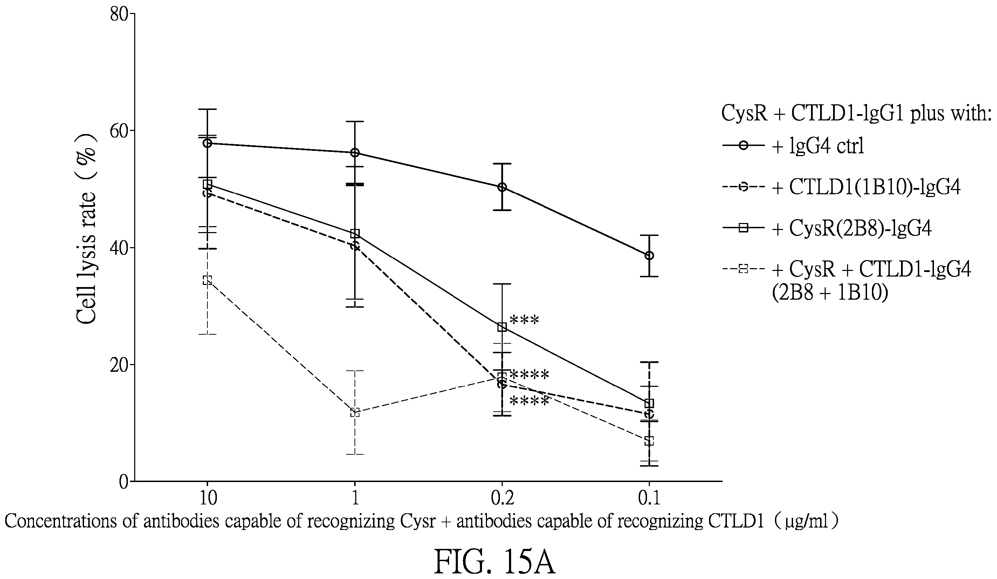

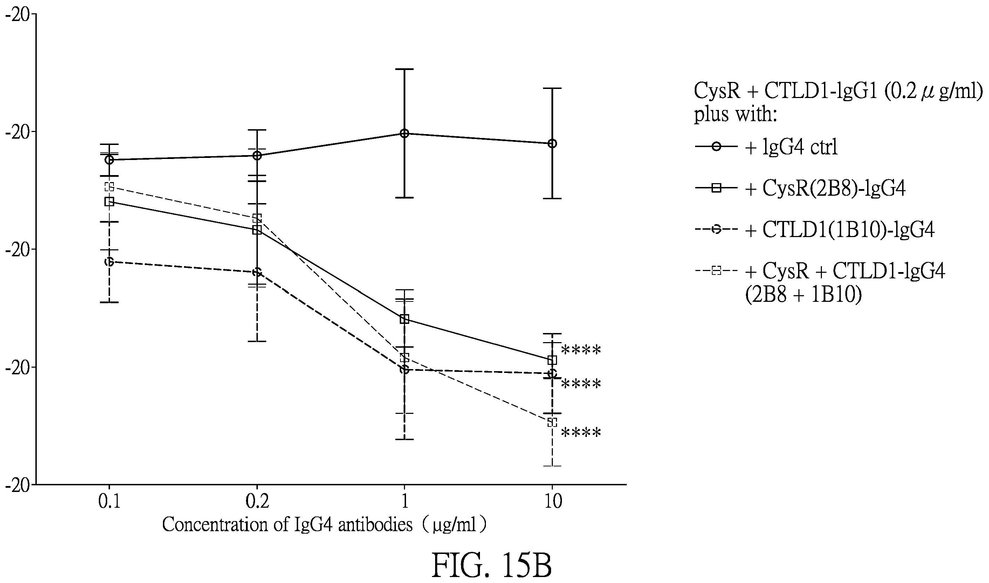

Results are shown in FIG. 14, FIG. 15A, and FIG. 15B. When the anti-PLA2R monoclonal antibody of the IgG1 subclass or the IgG3 subclass binds to the CTLD1 domain and the CysR domain of the PLA2R, the CDC induction is significantly enhanced. However, when the subclass of the anti-PLA2R monoclonal antibody is changed from IgG1 or IgG3 to IgG4, the cytotoxicity of said antibody is instantly and significantly reduced. Such result indicates that an antibody of the igG4 subclass does not have the activity to induce the CDC response, and the anti-PLA2R monoclonal antibody relies on the Fc fragment of the IgG1 subclass or the IgG3 subclass to induce the CDC response. Based on such result, a novel treatment strategy for membranous nephropathy can be provided. For example, an Fc-inactivated anti-PLA2R monoclonal antibody (i.e., not having the activity to induce the CDC response) can be used as an antagonist to interfere with binding of an autoantibody and the PLA2R, thereby blocking occurrence of the CDC induction and the cell lysis.

### [Embodiment 4 Evaluation on Use of Antagonist to Block CDC Induction of Anti-PLA2R Autoantibody]

### 4.1 Analysis of Suppression of CDC Induction by Use of Fab Fragments of Anti-PLA2R Monoclonal Antibody

The antibody engineering is employed to capture Fab fragments of the anti-PLA2R monoclonal antibodies mentioned above, such as the Fab fragments of the antibody 1B10 and the antibody 2B8. After the podocytes overexpressing PLA2R are seeded and cultured overnight in the 96-well flat-bottomed white plate (5 x 10³ cells/well), the RPMI medium containing 2% of FBS (100 µl per well) is selected for medium replacement, and the Fab fragments (132 nM) of the anti-PLA2R monoclonal antibody are added for incubation at 37°C for 1 hour. Then, IgG (15%) of the MN patient is added for incubation at 37°C for 1 hour, 5% of the baby rabbit complement (brand: Cedarlane) is added into each well for incubation at 37°C for 4 hours, and 100 µL of the CellTiter-Glo reagent is further added and incubated with shaking at 70 rpm for 15 minutes at the room temperature. Finally, the plate reader (brand: PerkinElmer) is used for measurement of the luminescence value, and the cell viability (%) and the cytotoxicity (%) are measured.

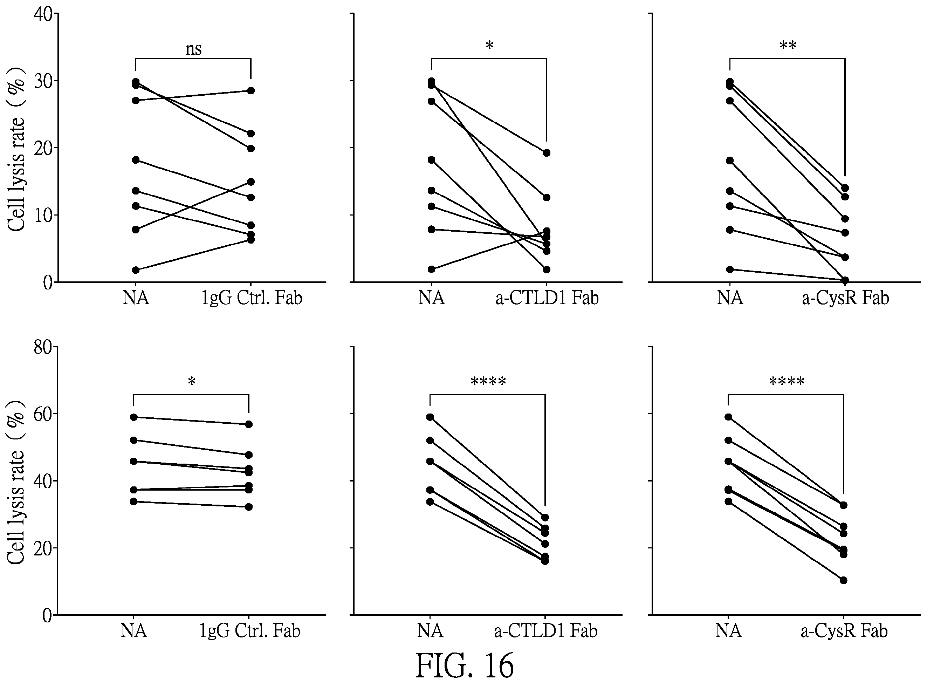

Results are shown in FIG. 16. Addition of the Fab fragments of the anti-PLA2R monoclonal antibody (e.g., α-CTLD1 Fab as labeled in the drawing is an Fab fragment capable of recognizing CTLD1 (binding to CLTD1), and α-CysR Fab as labeled in the drawing is an Fab fragment capable of recognizing CysR (binding to CysR)) can significantly reduce the CDC response induced by an anti-PLA2R autoantibody of pMN patients, thereby reducing occurrence of the cell lysis. Such results indicate that the Fab fragments of the anti-PLA2R monoclonal antibody that are capable of binding to the CTLD1 domain or the CysR domain of the PLA2R can act as a competitive antagonist to compete with the autoantibody of the pMN patients and block binding of the autoantibody of the pMN patients and the PLA2R, thereby slowing damage to the podocytes caused by the autoantibody of the pMN patients.

### 4.2 Analysis of Suppression of CDC Induction by Use of CTLD1 Recombinant Protein and CysR Recombinant Protein

A CTLD1 recombinant protein and a CysR recombinant protein (which are encoded by nucleic acid sequences as shown in Table 11) are manufactured. After the podocytes overexpressing PLA2R are seeded and cultured overnight in the 96-well flat-bottomed white plate (5 x 10³ cells/well), the RPMI medium containing 2% of FBS (100 µl per well) is selected for medium replacement, and the CTLD1 recombinant protein and the CysR recombinant protein (132 nM) are added for incubation at 37°C for 1 hour. Then, IgG (15%) of the pMN patient is added for incubation at 37°C for 1 hour, 5% of the baby rabbit complement (brand: Cedarlane) is added into each well for incubation at 37°C for 4 hours, and 100 µL of the CellTiter-Glo reagent is further added and incubated with shaking at 70 rpm for 15 minutes at the room temperature. Finally, the plate reader (brand: PerkinElmer) is used for measurement of the luminescence value, and the cell viability and the cytotoxicity are measured.

**Table 11**

| | Sequence | SEQ ID No: |
|---|---|---|
| CTLD1 recombinant protein | GATATCGAGGGTGTGGCGGCGGCGCTTACCCCCG AGCGGCTCCTGGAGTGGCAGGATAAAGGAATATT TGTTATCCAAAGTGAGAGTCTCAAGAAATGCATTC AAGCAGGTAAATCGGTTCTGACCCTGGAGAACTG CAAGCAAGCAAACAAGCACATGCTGTGGAAATG GGTTTCAAACCATGGCCTCTTTAACATAGGAGGCA GTGGTTGCCTGGGCCTGAATTTCTCCGCCCCAGA GCAGCCATTAAGCTTATATGAATGTGACTCCACCC TCGTTTCCTTACGGTGGCGCTGTAACAGGAAGAT GATCACAGGCCCGCTGCAGTACTCTGTCCAGGTG GCGCATGACAACACAGTGGTGGCCTCACGGAAGT ATATTCATAAGTGGATTTCTTATGGGTCAGGTGGT GGAGACATTTGTGAATAT | 190 |
| CysR recombinant protein | GATATCGAGGGTGTGGCGGCGGCGCTTACCCCCG AGCGGCTCCTGGAGTGGCAGGATAAAGGAATATT TGTTATCCAAAGTGAGAGTCTCAAGAAATGCATTC AAGCAGGTAAATCGGTTCTGACCCTGGAGAACTG CAAGCAAGCAAACAAGCACATGCTGTGGAAATG GGTTTCAAACCATGGCCTCTTTAACATAGGAGGCA GTGGTTGCCTGGGCCTGAATTTCTCCGCCCCAGA GCAGCCATTAAGCTTATATGAATGTGACTCCACCC | 191 |
| | TCGTTTCCTTACGGTGGCGCTGTAACAGGAAGAT GATCACAGGCCCGCTGCAGTACTCTGTCCAGGTG GCGCATGACAACACAGTGGTGGCCTCACGGAAGT ATATTCATAAGTGGATTTCTTATGGGTCAGGTGGT GGAGACATTTGTGAATAT | |

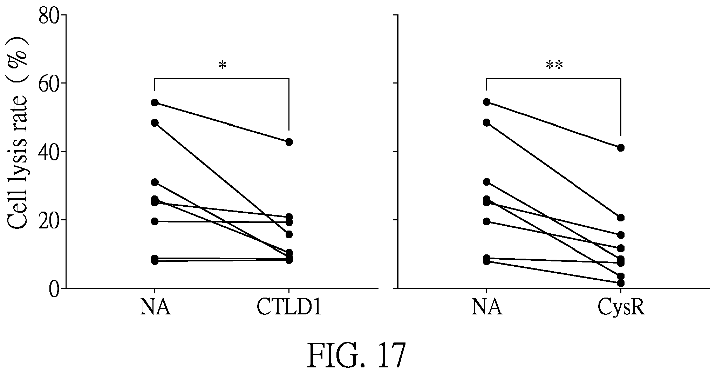

Results are shown in FIG. 17. Addition of the CTLD1 recombinant protein (labeled as CTLD1 in the drawing) or the CysR recombinant protein (labeled as CysR in the drawing) can significantly reduce the CDC response induced by the anti-PLA2R autoantibody of the pMN patients, thereby reducing occurrence of the cell lysis. Such results indicate that the CTLD1 recombinant protein or the CysR recombinant protein can act as the antagonist to bind to the autoantibody of the pMN patients and block the CDC induced by binding of the autoantibody and the PLA2R of the podocytes, thereby slowing damage to the podocytes caused by the autoantibody of the pMN patients.

### [Beneficial Effects of the Embodiments]

In conclusion, a novel treatment strategy and a novel pharmaceutical composition for treating the membranous nephropathy are provided. After much experimentation, it is observed that the anti-PLA2R autoantibody needs to bind to the CTLD1 domain or the CysR domain of the PLA2R, and relies on the Fc fragment of IgG1 or the Fc fragment of IgG3 to induce the complement-dependent cytotoxicity.

Accordingly, the novel treatment strategy for the membranous nephropathy provided in the present invention includes using the antagonist to interfere with binding of the anti-PLA2R autoantibody and the PLA2R in the cells, so as to suppress the cytotoxicity of the anti-PLA2R autoantibody.

In the present invention, by virtue of "the antagonist being the antibody and being configured to bind to the CTLD1 domain or the CysR domain of the PLA2R" and/or "the antagonist being the recombinant protein and being configured to bind to the anti-PLA2R autoantibody," the antagonist can block binding of the anti-PLA2R autoantibody and the PLA2R in the cells (e.g., the podocytes), thereby blocking the complement-dependent cytotoxicity induced by the anti-PLA2R autoantibody and reducing cell damage.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated.

## Claims

1. An antagonist for use in the treatment of membranous nephropathy, **characterized in that** the membranous nephropathy is an autoantibody-mediated membranous nephropathy, the antagonist is an antibody, and the antibody is configured to bind to a C-type lectin-like domain 1 (CTLD1) domain or a cysteine-rich (CysR) domain of a phospholipase A2 receptor (PLA2R).

2. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:151, the L-CDR2 contains an amino acid sequence set forth in SEQ ID NO:152, the L-CDR3 contains an amino acid sequence set forth in SEQ ID NO:153, the H-CDR1 contains an amino acid sequence set forth in SEQ ID NO:112, the H-CDR2 contains an amino acid sequence set forth in SEQ ID NO:113, and the H-CDR3 contains an amino acid sequence set forth in SEQ ID NO:114.

3. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:154, the L-CDR2 contains an amino acid sequence set forth in SEQ ID NO:155, the L-CDR3 contains an amino acid sequence set forth in SEQ ID NO:156, the H-CDR1 contains an amino acid sequence set forth in SEQ ID NO:115, the H-CDR2 contains an amino acid sequence set forth in SEQ ID NO:116, and the H-CDR3 contains an amino acid sequence set forth in SEQ ID NO:117.

4. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:157, the L-CDR2 contains an amino acid sequence set forth in SEQ ID NO: 158, the L-CDR3 contains an amino acid sequence set forth in SEQ ID NO:159, the H-CDR1 contains an amino acid sequence set forth in SEQ ID NO:118, the H-CDR2 contains an amino acid sequence set forth in SEQ ID NO:119, and the H-CDR3 contains an amino acid sequence set forth in SEQ ID NO:120.

5. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:160, the L-CDR2 contains an amino acid sequence set forth in SEQ ID NO:161, the L-CDR3 contains an amino acid sequence set forth in SEQ ID NO:162, the H-CDR1 contains an amino acid sequence set forth in SEQ ID NO:121, the H-CDR2 contains an amino acid sequence set forth in SEQ ID NO:122, and the H-CDR3 contains an amino acid sequence set forth in SEQ ID NO:123.

6. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:163, the L-CDR2 contains an amino acid sequence set forth in SEQ ID NO:164, the L-CDR3 contains an amino acid sequence set forth in SEQ ID NO:165, the H-CDR1 contains an amino acid sequence set forth in SEQ ID NO:124, the H-CDR2 contains an amino acid sequence set forth in SEQ ID NO:125, and the H-CDR3 contains an amino acid sequence set forth in SEQ ID NO:126.

7. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:166, the L-CDR2 contains an amino acid sequence set forth in SEQ ID NO:167, the L-CDR3 contains an amino acid sequence set forth in SEQ ID NO:168, the H-CDR1 contains an amino acid sequence set forth in SEQ ID NO:127, the H-CDR2 contains an amino acid sequence set forth in SEQ ID NO:128, and the H-CDR3 contains an amino acid sequence set forth in SEQ ID NO:129.

8. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:169, the L-CDR2 contains an amino acid sequence set forth in SEQ ID NO:170, the L-CDR3 contains an amino acid sequence set forth in SEQ ID NO:171, the H-CDR1 contains an amino acid sequence set forth in SEQ ID NO:130, the H-CDR2 contains an amino acid sequence set forth in SEQ ID NO:131, and the H-CDR3 contains an amino acid sequence set forth in SEQ ID NO:132.

9. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:172, the L-CDR2 contains an amino acid sequence set forth in SEQ ID NO:173, the L-CDR3 contains an amino acid sequence set forth in SEQ ID NO:174, the H-CDR1 contains an amino acid sequence set forth in SEQ ID NO:133, the H-CDR2 contains an amino acid sequence set forth in SEQ ID NO:134, and the H-CDR3 contains an amino acid sequence set forth in SEQ ID NO:135.

10. The antagonist for use in the treatment of membranous nephropathy according to claim **1,** wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:175, the L-CDR2 contains an amino acid sequence set forth in SEQ ID NO:176, the L-CDR3 contains an amino acid sequence set forth in SEQ ID NO:177, the H-CDR1 contains an amino acid sequence set forth in SEQ ID NO:136, the H-CDR2 contains an amino acid sequence set forth in SEQ ID NO:137, and the H-CDR3 contains an amino acid sequence set forth in SEQ ID NO:138.

11. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:178, the L-CDR2 contains an amino acid sequence set forth in SEQ ID NO:179, the L-CDR3 contains an amino acid sequence set forth in SEQ ID NO:180, the H-CDR1 contains an amino acid sequence set forth in SEQ ID NO:139, the H-CDR2 contains an amino acid sequence set forth in SEQ ID NO:140, and the H-CDR3 contains an amino acid sequence set forth in SEQ ID NO:141.

12. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:181, the L-CDR2 contains an amino acid sequence set forth in SEQ ID NO:182, the L-CDR3 contains an amino acid sequence set forth in SEQ ID NO:183, the H-CDR1 contains an amino acid sequence set forth in SEQ ID NO:142, the H-CDR2 contains an amino acid sequence set forth in SEQ ID NO:143, and the H-CDR3 contains an amino acid sequence set forth in SEQ ID NO:144.

13. The antagonist for use in the treatment of membranous nephropathy according to claim 1, wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:184, the L-CDR2 contains an amino acid sequence set forth in SEQ ID NO:185, the L-CDR3 contains an amino acid sequence set forth in SEQ ID NO:186, the H-CDR1 contains an amino acid sequence set forth in SEQ ID NO:145, the H-CDR2 contains an amino acid sequence set forth in SEQ ID NO: 146, and the H-CDR3 contains an amino acid sequence set forth in SEQ ID NO:147.

14. The antagonist for use in the treatment of membranous nephropathy according to claim **1,** wherein the antibody contains light chains and heavy chains, the light chain contains complementarity-determining regions L-CDR1, L-CDR2, and L-CDR3, and the heavy chain contains complementarity-determining regions H-CDR1, H-CDR2, and H-CDR3; and wherein the L-CDR1 contains an amino acid sequence set forth in SEQ ID NO:187, the L-CDR2 contains an amino acid sequence set forth in SEQ ID NO:188, the L-CDR3 contains an amino acid sequence set forth in SEQ ID NO:189, the H-CDR1 contains an amino acid sequence set forth in SEQ ID NO:148, the H-CDR2 contains an amino acid sequence set forth in SEQ ID NO:149, and the H-CDR3 contains an amino acid sequence set forth in SEQ ID NO:150.

15. The antagonist for use in the treatment of membranous nephropathy according to claim 1, wherein the antibody is a fragment antigen-binding (Fab) fragment.

16. An antagonist for use in the treatment of membranous nephropathy, **characterized in that** the membranous nephropathy is an autoantibody-mediated membranous nephropathy, the antagonist is a recombinant protein, and the recombinant protein is configured to bind to an anti-PLA2R autoantibody.

17. The antagonist for use in the treatment of membranous nephropathy according to claim 16, wherein the recombinant protein contains a peptide encoded by a nucleic acid sequence set forth in SEQ ID NO: 190 or 191.

18. An antagonist, **characterized in that** the antagonist includes the antibody as claimed in any one of claims 1-15.
